(19) Europäisches Patentamt
European Patent Office
Office européen des brevets

(11) Numéro de publication: **0 291 401 B1**

# (12) FASCICULE DE BREVET EUROPEEN

(45) Date de publication du fascicule du brevet: **03.10.90**

(51) Int. Cl.⁵: **C07C 7/11**

(21) Numéro de dépôt: **88401131.3**

(22) Date de dépôt: **10.05.88**

(54) **Procédé cryogénique de désulfuration sélective et de dégazolinage simultanés d'un mélange gazeux consistant principalement en méthane et renfermant également H2S ainsi que des hydrocarbures en C2 et plus.**

(30) Priorité: **15.05.87 FR 8706843**

(43) Date de publication de la demande: **17.11.88 Bulletin 88/46**

(45) Mention de la délivrance du brevet: **03.10.90 Bulletin 90/40**

(84) Etats contractants désignés: **AT DE ES FR GB GR IT NL**

(56) Documents cités:
**GB-A- 2 133 309**
**GB-A- 2 133 717**

(73) Titulaire: **SOCIETE NATIONALE ELF AQUITAINE (PRODUCTION), Tour Elf 2, Place de la Coupole La Défense 6, F-92400 Courbevoie(FR)**

(72) Inventeur: **Blanc, Claude, 24, rue de Bagnères, F-64000 Pau(FR)**
Inventeur: **Paradowski, Henri, 32, rue Serpente, F-95000 Cergy(FR)**

(74) Mandataire: **Boillot, Marc, SOCIETE NATIONALE ELF AQUITAINE Division Propriété Industrielie Tour Elf, F-92078 Paris la Défense Cédex 45(FR)**

EP 0 291 401 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention concerne un procédé cryogénique de désulfuration sélective et de dégazolinage simultanés d'un mélange gazeux consistant principalement en méthane et renfermant également $H_2S$, des hydrocarbures en $C_2$ et plus et éventuellement un ou plusieurs composés gazeux choisis parmi les gaz inertes, $H_2O$, $CO_2$, COS et des mercaptans, ledit mélange gazeux étant sous une pression absolue supérieure à 0,5 MPa. Le procédé suivant l'invention permet de séparer directement un mélange gazeux du type précité en trois composantes, à savoir :

- un gaz traité consistant principalement en méthane et dont la pression partielle en $H_2S$ est inférieure à 65 Pa et la pression partielle globale en composés soufrés, lorsque le mélange gazeux à traiter renferme COS et/ou des mercaptans en plus d'$H_2S$, est inférieure à 260 Pa,
- une coupe d'hydrocarbures lourds contenant au moins 80 % molaire des hydrocarbures en $C_3$ et plus présents dans le mélange gazeux à traiter, et
- un courant de gaz acide consistant en $H_2S$ éventuellement mélangés avec une quantité plus ou moins importante de composés acides $CO_2$, COS et mercaptans, lorsque lesdits composés sont présents dans le mélange gazeux à traiter, ledit courant de gaz acide d'une part contenant moins de 5 % molaire d'hydrocarbures, exprimés en équivalent méthane, par rapport à l'$H_2S$ et autres composés acides et plus de 80 % de l'$H_2S$ contenu dans le mélange gazeux à traiter et d'autre part ayant un rapport molaire $H_2S$ : $CO_2$ supérieur au rapport correspondant dans le mélange gazeux à traiter.

On connaît plusieurs procédés, utilisés industriellement, pour le traitement de mélanges gazeux tels que définis plus haut et dont les principaux exemples sont représentés par les divers gaz naturels, qui comportent une opération de désacidification, c'est-à-dire une élimination de l'$H_2S$ et autres gaz acides, et une opération de dégazolinage, c'est-à-dire une séparation des hydrocarbures lourds par exemple en $C_3$ et plus, du mélange gazeux et permettent de réaliser le fractionnement dudit mélange gazeux en les trois composantes mentionnées ci-dessus. Ces opérations de désacidification et de dégazolinage sont généralement mises en oeuvre de manière séparée et font partie d'une succession d'opérations réalisées sur le mélange gazeux à traiter et comportant principalement une élimination des gaz acides, un séchage, une adsorption de l'eau sur un solide approprié tel qu'un tamis moléculaire, une séparation par distillation cryogénique entre -30°C et -90°C associée ou non à une extraction par un solvant afin d'obtenir la coupe de liquide de gaz naturel, et enfin un réchauffage du gaz traité jusqu'à la température ambiante pour, généralement, alimenter le réseau de gaz commercial.

Dans un tel schéma de traitement d'un mélange gazeux du type gaz naturel renfermant les constituants précités, l'abaissement de la température du mélange gazeux est imposée par la seule production de la coupe de liquide de gaz naturel, aucune autre opération n'étant effectuée à ce niveau de température.

Dans ce type de schéma de traitement, la réalisation en série d'opérations, qui s'appuient sur des principes très différents et sont conduites à des niveaux de température divers, présente de sérieux inconvénients. Il n'y a que peu de possibilité d'intégration thermique, ce qui rend ledit schéma de traitement extrêmement onéreux au plan énergétique et au plan des investissements. En outre, au niveau de l'étape d'élimination des composés gazeux acides, qui constitue généralement la première étape dudit schéma de traitement et consiste habituellement à laver le mélange gazeux à traiter, à contre-courant, à l'aide d'un solvant des composés gazeux acides sélectif ou non de l'$H_2S$ et régénérable comme une solution aqueuse d'une alcanolamine ou de carbonate de potassium ou encore un solvant organique, des contraintes apparaissent dans le choix du solvant dès que le mélange gazeux à traiter renferme des quantités même assez faibles d'hydrocarbures en $C_3$ et plus.

En effet, les solvants organiques disponibles industriellement et susceptibles de dissoudre efficacement $H_2S$ et les autres composés acides tels que $CO_2$, COS et mercaptans, par exemple carbonate de propylène, méthanol, N-méthylpyrrolidone, diméthyléther d'éthylène glycol ou de polyéthylène glycols en $C_4$ à $C_{12}$, ne sont utilisables que si le mélange gazeux à traiter ne contient que de très faibles quantités desdits hydrocarbures. Dans le cas contraire ces hydrocarbures en $C_3$ et plus sont en grande partie absorbés par le solvant organique et se retrouvent dans le courant de gaz acide renfermant $H_2S$ produit au cours de la régénération du solvant, ce qui entraîne, lors de l'utilisation habituelle dudit courant de gaz acide dans une usine à soufre, un fonctionnement défectueux de cette dernière et il est alors préférable d'écarter l'emploi de solvants organiques au profit de solvants consistant en solutions aqueuses d'amines, notamment alcanolamines, ou de carbonate de potassium.

On connaît également des procédés de traitement de mélanges gazeux du type des gaz naturels, qui permettent de réaliser simultanément l'élimination des gaz acides contenus dans le mélange gazeux et la production d'hydrocarbures gazeux et d'hydrocarbures liquides et dont le type est le procédé connu sous le nom de procédé RYAN-HOLMES et décrit, notamment, par J. RYAN et F. SCHAFFERT dans la revue CHEMICAL ENGINEERING PROGRESS, Octobre 1984, pages 53 à 56. Dans un tel procédé, le gaz naturel à traiter, après avoir été déshydraté de manière conventionnelle puis réfrigéré, est soumis à une distillation à basse température mise en oeuvre en trois ou quatre étapes successives. Dans le mode de réalisation en trois étapes, le gaz naturel déshydraté et réfrigéré est séparé, dans une première colonne (déméthaniseur) en tête de laquelle est injecté un additif consistant en une fraction liquide d'hydrocarbures en $C_4$ et plus, en une phase gazeuse renfermant le méthane et les composés plus légers et une fraction liquide contenant les hydrocarbures en $C_2$ et plus et les gaz acides.

Cette fraction liquide est séparée, dans une deuxième colonne (dé-éthaniseur) dans laquelle on introduit également une certaine quantité de l'additif, en une fraction de tête consistant en $CO_2$ et en une fraction de queue renfermant les hydrocarbures en $C_2$ et plus et l'$H_2S$. Ladite fraction de queue est ensuite séparée, dans une troisième colonne, en une fraction de tête consistant en une coupe liquide d'hydrocarbures en $C_2$ à $C_4$ renfermant l'$H_2S$ et en une fraction de queue consistant en une coupe liquide d'hydrocarbures en $C_4$ et plus, qui contient la majeure partie des butanes et les hydrocarbures supérieurs présents dans le gaz naturel traité et dont on prélève la quantité appropriée pour constituer l'additif injecté dans les première et seconde colonnes. L'utilisation de cet additif évite la cristallisation de $CO_2$ en tête du déméthaniseur et assure la rupture de l'azéotrope qui se forme entre l'éthane et $CO_2$ et facilite la séparation de ces composés dans le dé-éthaniseur. La coupe liquide d'hydrocarbures en $C_2$ à $C_4$ renfermant l'$H_2S$ est ensuite soumise à un lavage au moyen d'une solution aqueuse d'amine pour fournir une coupe liquide purifiée d'hydrocarbures en $C_2$ à $C_4$ et un courant de gaz acide renfermant $H_2S$ et éventuellement $CO_2$ utilisable pour la production de soufre dans les unités CLAUS.

Dans un tel procédé, la nécessité d'avoir recours à un additif d'une part pour éviter la cristallisation du $CO_2$ en tête du déméthaniseur et d'autre part pour rompre l'azéotrope entre l'éthane et $CO_2$ dans le déethaniseur rend la mise en oeuvre du procédé plus complexe et de ce fait plus onéreuse.

On sait encore que l'on peut éliminer les gaz acides, et notamment $H_2S$, contenus dans un gaz naturel par lavage à l'aide d'un solvant comme le méthanol, en opérant à des températures généralement inférieures à -10°C. En particulier dans la citation FR-A-2550956, on propose un procédé de ce type dans lequel on effectue un lavage du gaz naturel par le méthanol froid en opérant dans une pluralité de zones de lavage, qui sont disposées en série et dont la température d'opération va préférentiellement décroissant d'une zone à la zone immédiatement en aval. Le gaz naturel à traiter, injecté à l'entrée de la première zone, passe d'une zone à la suivante pour sortir de la dernière zone fortement appauvri en gaz acides tandis que du méthanol froid est injecté dans chaque zone et séparé du gaz naturel à la sortie de cette zone. Le méthanol introduit dans chaque zone autre que la dernière est le méthanol usagé séparé à la sortie de la zone immédiatement en aval tandis que le méthanol séparé de la première zone sert, après régénération, à alimenter la dernière zone. Avant la phase de lavage au méthanol, le gaz naturel peut être soumis à une purification par perméation en vue d'éliminer, entre autres, une certaine quantité de gaz acides, tandis que le gaz naturel issu du lavage au méthanol et pratiquement exempt d'$H_2S$ peut être soumis à un traitement de liquéfaction. Le gaz naturel purifié peut être également soumis à un traitement conventionnel de dégazolinage, sur lequel la citation ne donne aucune information. En outre, cette citation ne fournit aucun renseignement sur la teneur en hydrocarbures du gaz acide issu de la régénération du solvant, dont on sait qu'elle constitue un élément capital pour le bon fonctionnement des usines à soufre traitant ledit gaz acide.

L'invention propose un procédé cryogénique de désulfuration sélective et de dégazolinage simultanés de mélange gazeux du type des gaz naturels, c'est-à-dire de mélanges gazeux sous une pression absolue supérieure à 0,5 MPa, qui consistent principalement en méthane et renferment également $H_2S$, des hydrocarbures en $C_2$ et plus et éventuellement un ou plusieurs composés gazeux choisis parmi les gaz inertes, $H_2O$, $CO_2$, COS et des mercaptans, ledit procédé permettant d'atteindre plus facilement et à moindre coût, en comparaison aux procédés connus, l'objectif d'une séparation du mélange gazeux en les trois composantes, à savoir gaz traité consistant principalement en méthane, coupe liquide d'hydrocarbures lourds et courant de gaz acides renfermant $H_2S$, qui ont les spécifications définies plus haut.

Le procédé suivant l'invention est du type dans lequel on met le mélange gazeux en contact dans une zone de lavage avec un solvant qui dissout préférentiellement $H_2S$, COS et les mercaptans et seulement une partie du $CO_2$ et qui possède d'une part une température d'ébullition à la pression atmosphérique supérieure à 40°C et d'autre part une viscosité à -40°C inférieure à 0,1 Pa.s et l'on effectue une régénération du solvant renfermant l'$H_2S$ et les autres composés absorbés et il se caractérise en ce que ladite mise en contact du mélange gazeux avec le solvant est réalisée à une température suffisamment basse et avec un rapport des débits massiques de mélange gazeux à traiter et de solvant et un nombre d'étages théoriques de lavage tels que l'on produise d'une part un gaz traité consistant principalement en méthane et dont la pression partielle en $H_2S$ est inférieure à 65 Pa et, lorsqu'en plus d'$H_2S$ le mélange gazeux à traiter renferme également COS et/ou des mercaptans, la pression partielle globale des composés soufrés est inférieure à 260 Pa et d'autre part une phase liquide, appelée solvant riche, composée du solvant enrichi en $H_2S$ et autres composés absorbés et d'une fraction d'hydrocarbures condensés représentant au moins 80% molaire des hydrocarbures en $C_3$ et plus présents dans le mélange gazeux à traiter, on soumet le solvant riche à un traitement de déméthanisation au moins partielle pour produire une phase liquide appauvrie en méthane et appelée solvant riche déméthanisé et une phase gazeuse riche en méthane, qui peut être éventuellement réunie au mélange gazeux à traiter avant la mise en contact de ce dernier avec le solvant, on refroidit le solvant riche déméthanisé à une température suffisamment inférieure à la température régnant dans la zone de lavage pour provoquer une démixtion dudit solvant déméthanisé en une fraction liquide inférieure appelée solvant riche purifié, qui renferme les composés acides $H_2S$ et le cas échéant COS, $CO_2$ et mercaptans dissous par le solvant et possède une teneur en hydrocarbures, exprimée en équivalent méthane, inférieure à 5% molaire de la quantité de composés acides dissous et en une fraction liquide supérieure appelée coupe primaire d'hydrocarbures lourds et formée du reste des hydrocarbures qui étaient présents dans le solvant riche déméthanisé, on sépare la

coupe primaire d'hydrocarbures lourds du solvant riche purifié et l'on soumet ledit solvant riche purifié au traitement de régénération pour produire un courant de gaz acide renfermant la quasi totalité de l'$H_2S$ et autres composés acides éventuels présents dans le solvant riche purifié et contenant, exprimé en équivalent méthane, moins de 5% molaire d'hydrocarbures par rapport aux composés acides désorbés et un solvant régénéré que l'on recycle vers la zone de lavage.

Par "équivalent méthane", on désigne suivant l'invention autant de pseudo-molécules à un seul atome de carbone qu'il y a d'atomes de carbone dans la molécule considérée d'hydrocarbure.

Le solvant, qui est défini généralement ci-dessus pour la mise en contact avec le mélange gazeux à traiter au fins d'absorption de l'$H_2S$ et autres composés COS et mercaptans, possède de préférence une viscosité inférieure à 0,05 Pa.s.

Le solvant utilisé suivant l'invention est sélectif de l'$H_2S$, c'est-à-dire qu'il possède une capacité d'absorption substantiellement plus élevée pour l'$H_2S$ et autres composés soufrés précités que pour le $CO_2$ et conduit de ce fait à l'obtention, dans le courant de gaz acide produit lors de la régénération du solvant, d'un rapport molaire $H_2S$ et autres composés soufrés : $CO_2$ supérieur au rapport correspondant dans le mélange gazeux à traiter.

Le solvant suivant l'invention peut consister en particulier en un ou plusieurs absorbants liquides utilisés sous forme anhydre ou en mélange avec de l'eau, le ou lesdits solvants étant choisis parmi les amides de formules

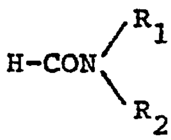 et 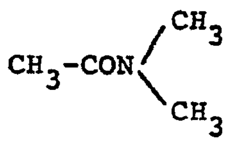, les aldéhydes de formule 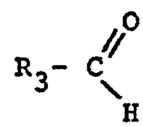

les acétals de formule 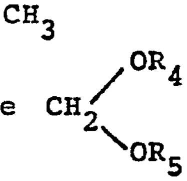 et 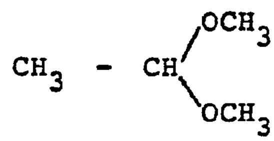, les

esters de formules 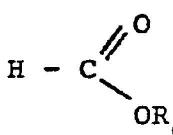 et 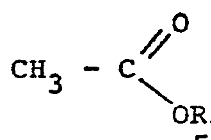, les alcanols

en $C_1$ à $C_4$, les diéthers de formule $CH3\ O-[C_2H_4O]_n-CH_3$,
les diéthers alcools de formule $R_9O - C_2H_4 - O - C_2H_4 - OH$,

les lactones de formule 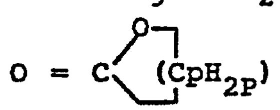

et le carbonate de propylène, avec dans ces formules $R_1$ et $R_2$, identiques ou différents, désignant un atome d'hydrogène ou un radical alcoyle en $C_1$ ou $C_2$, $R_3$ étant un radical alcoyle en $C_3$ ou $C_4$, $R_4$ et $R_5$, identiques ou différents, représentant un radical alcoyle en $C_1$ à $C_3$, $R_6$ étant un radical alcoyle en $C_2$ à $C_4$ ou un radical

$$-[C_2H_4O]_n-R_8$$

avec $R_8$ désignant un radical alcoyle en $C_1$ ou $C_2$ et n étant égal à 1 ou 2, $R_7$ étant un radical alcoyle en $C_1$ ou $C_2$ ou un radical

$$[C_2H_4O]_n-R_8,$$

$R_9$ désignant un radical alcoyle en $C_1$ à $C_4$ et p étant un nombre entier allant de 2 à 4.

Des exemples non limitatifs d'absorbants organiques liquides répondant aux formules ci-dessus sont tels que N,N-diméthylformamide, N,N-diméthylacétamide, diméthoxyméthane, diéthoxyméthane, diméthoxy-1,1 éthane, méthanol, éthanol, diméthyléther de l'éthylène glycol, diméthyléther du diéthylèneglycol, monométhyléther de l'éthylèneglycol, butyrolactone, propiolactone et carbonate de propylène.

La température de mise en contact du mélange gazeux à traiter avec le solvant, dans la zone de lavage, est de préférence comprise entre 0°C et -45°C.

La zone de lavage consiste en une ou plusieurs colonnes de lavage renfermant chacune avantageusement au moins environ 10 et de préférence au moins 20 étages théoriques de lavage, lesdites colonnes étant, par exemple, du type des colonnes à plateaux ou encore des colonnes à garnissage. Avantageusement on maintient la température dans chacune des colonnes de lavage, de préférence à ± 5°C près, par échange indirect de chaleur, effectué en un ou plusieurs points de la colonne considérée, entre le milieu fluide contenu dans cette colonne et un fluide réfrigérant.

Suivant un mode particulier de mise en oeuvre du procédé suivant l'invention, le mélange gazeux à traiter est soumis, préalablement à sa mise en contact avec le solvant, à une réfrigération jusqu'à une température comprise entre 0°C et -30°C pour produire d'une part une phase liquide appelée condensats contenant principalement des hydrocarbures en $C_3$ et plus et $H_2S$ et d'autre part une phase gazeuse dite mélange gazeux prétraité, puis l'on met ledit mélange gazeux prétraité en contact avec le solvant dans la zone de lavage et l'on réunit le solvant issu de la zone de lavage aux condensats issus de l'étape de réfrigération pour constituer le solvant riche que l'on soumet au traitement de déméthanisation.

Suivant un autre mode particulier de mise en oeuvre du procédé suivant l'invention, le mélange gazeux à traiter, préalablement à sa mise en contact avec le solvant, est soumis à une réfrigération jusqu'à une température comprise entre 0°C et -30°C pour produire d'une part une phase liquide appelée condensats contenant principalement des hydrocarbures en $C_3$ et plus et $H_2S$ et d'autre part une phase gazeuse dite mélange gazeux prétraité, puis l'on met ledit mélange gazeux prétraité en contact avec le solvant dans la zone de lavage et l'on soumet chacune des phases liquides constituées par le solvant issu de la zone de lavage et les condensats issus de l'étape de réfrigération à un traitement séparé de déméthanisation, et l'on réunit le solvant et les condensats issus des traitements séparés de déméthanisation pour constituer le solvant riche déméthanisé. De préférence, les phases gazeuses produites au cours des traitements de déméthanisation sont recyclées, séparément ou en mélange, vers le mélange gazeux à traiter en amont de l'étape de réfrigération.

Avantageusement le traitement de déméthanisation appliqué au solvant riche et aux condensats est réalisé en deux étapes à savoir une première étape dans laquelle le fluide à déméthaniser, à savoir solvant riche ou condensats, est soumis à une première détente à une pression intermédiaire propre à libérer une fraction importante du méthane dissous dans ledit fluide à déméthaniser et à produire un premier gaz riche en méthane et un fluide prédéméthanisé et une seconde étape dans laquelle le fluide prédéméthanisé est soumis à une seconde détente puis à une distillation de manière à produire un second gaz riche en méthane et un fluide déméthanisé, le second gaz riche en méthane étant comprimé jusqu'à la pression du premier gaz riche en méthane puis mélangé à ce dernier pour constituer une phase gazeuse riche en méthane, que l'on recycle éventuellement vers le mélange gazeux à traiter. De préférence, dans la seconde étape du traitement de déméthanisation, la distillation faisant suite à la seconde détente s'effectue par rebouillage réalisé, dans aux moins deux zones de rebouillage disposées en série, au moyen d'un fluide consistant soit en au moins une partie du mélange gazeux à traiter, prélevée sur ledit mélange gazeux avant sa mise en contact avec le solvant ou avant sa réfrigération, ou encore en une partie du solvant régénéré.

Avantageusement la température, inférieure à la température du solvant riche déméthanisé, à laquelle on refroidit ledit solvant pour provoquer sa démixtion est comprise entre -25°C et -80°C.

La régénération du solvant riche purifié peut être réalisée par tout traitement, par exemple détente et/ou distillation, permettant de libérer les composés gazeux dissous dans un liquide. En particulier le traitement de régénération du solvant riche purifié peut comporter une étape de régénération partielle comprenant une détente du solvant riche purifié jusqu'à une pression supérieure à 100 KPa et de préférence comprise entre 150 KPa et 300 KPa suivie d'au moins une vaporisation partielle dudit solvant détendu de manière à produire d'une part au moins une fraction de gaz acide et d'autre part un solvant semi-régénéré dont la température est au moins égale à 0°C et la pression, inférieure à la pression du solvant détendu, est au moins égale à 100 KPa, puis une étape de régénération totale du solvant semi-régénéré réalisée par distillation, notamment en faisant appel à un rebouillage, et produisant une fraction de gaz acide et du solvant régénéré, ledit solvant régénéré, étant recyclé, après refroidissement approprié, vers la zone de lavage tandis que les différentes fractions de gaz acide sont réunies pour former le courant de gaz acide dont la teneur en hydrocarbures, exprimée en équivalent méthane, est inférieure à 5 % molaire des composés acides.

Dans l'étape de régénération partielle du solvant riche purifié, la vaporisation partielle du solvant détendu est mise en oeuvre avantageusement en réalisant tout d'abord une vaporisation partielle primaire dudit solvant de manière à produire une fraction primaire de gaz acide et une phase liquide primaire dont la température est inférieure à 0°C puis en effectuant une vaporisation partielle secondaire de la phase liquide primaire de manière à produire une fraction secondaire de gaz acide et une phase liquide secondaire ayant une température au moins égale à 0°C et une pression, inférieure à la pression du solvant détendu, au moins égale à 100 KPa, ladite phase liquide secondaire constituant le solvant semi-régénéré que l'on traite dans l'étape de régénération totale, la fraction primaire de gaz acide étant réchauffée jusqu'à une température égale ou supérieure à 0°C puis réunie à la fraction secondaire de gaz acide et à la fraction de gaz acide résultant de la régénération totale du solvant semi-régénéré pour former le courant de gaz acide dont la teneur en hydrocrabures, exprimée en équivalent méthane, est inférieure à 5% molaire des composés acides.

La mise en oeuvre de la régénération totale du solvant semi-régénéré par distillation est réalisée dans une colonne comportant une pluralité d'étages théoriques avec soutirage du solvant régénéré en fond de colonne.

Dans ce cas, on peut diviser le solvant semi-régénéré en deux courants dont l'un est envoyé sur le plateau de tête de la colonne de distillation et l'autre alimente ladite colonne au niveau d'un étage théorique intermédiaire, après réchauffage à contre-courant avec le solvant régénéré soutiré de la colonne.

Avant sa régénération, le solvant riche purifié peut être soumis à un traitement complémentaire d'élimination des hydrocarbures, ledit traitement consistant à mettre en contact ledit solvant purifié, dans une zone primaire d'extraction liquide-liquide comportant, de préférence, au moins quatre étages théoriques d'extraction, avec un agent d'extraction principalement constitué d'hydrocarbures dont la masse moléculaire est supérieure à celle du n-pentane, de manière à séparer dudit solvant riche purifié une coupe secondaire liquide d'hydrocarbures, à réunir ladite coupe secondaire à la coupe primaire d'hydrocarbures lourds, puis à réchauffer et distiller le mélange ainsi obtenu pour produire la coupe d'hydrocarbures lourds contenant au moins 80% molaire des hydrocarbures en $C_3$ et plus présents dans le mélange gazeux à traiter et récupérer l'agent d'extraction, dont une partie principale est recyclée vers la zone primaire d'extraction liquide-liquide, après refroidissement jusqu'à une température sensiblement égale à celle atteinte lors de la démixtion du solvant riche déméthanisé.

Avantageusement le gaz traité issu de la zone de lavage est réfrigéré d'au moins 15°C de manière à produire un gaz traité réfrigéré et une coupe liquide qui est soumise au traitement de déméthanisation en mélange avec le solvant riche. On peut encore soumettre le gaz traité réfrigéré, dans une zone complémentaire d'extraction, à une extraction additionnelle au moyen de la partie de l'agent d'extraction non utilisée dans la zone d'extraction primaire, de manière à produire un gaz traité de pureté améliorée et une fraction liquide que l'on recycle vers la distillation du mélange des coupes primaire et secondaire d'hydrocarbures.

Avant tout traitement le mélange gazeux à traiter peut être soumis à une opération de séchage et de débenzolage par distillation et mise en contact avec du solvant anhydre, de manière à produire d'une part un mélange gazeux sec, dont la teneur en hydrocarbures aromatiques, notamment benzène, est inférieure à 0,1% en poids et d'autre part une coupe hydrocarbonée renfermant la plus grande partie des hydrocarbures aromatiques contenus dans le mélange gazeux à traiter et un liquide consistant en solvant renfermant de l'eau.

L'invention sera mieux comprise à la lecture de la description donnée ci-après de trois de ses modes de réalisation faisant appel aux installations représentées schématiquement sur les figures 1 à 4 du dessin annexé.

En se référant à la figure 1, le mélange gazeux à traiter arrivant par un conduit 1 subit une réfrigération, dans une zone 2 de réfrigération, jusqu'à une température comprise, par exemple, entre 0°C et -30°C et se sépare, dans un séparateur 2a, d'une part en une phase liquide appelée condensats et contenant principalement des hydrocarbures en $C_3$ et plus et de l'$H_2S$, qui est soutirée en fond du séparateur par un conduit 4, et d'autre part en une phase gazeuse, dite mélange gazeux prétraité, sortant en tête du séparateur par un conduit 3. Ledit mélangé gazeux prétraité est introduit à la partie inférieure d'une colonne 5 de lavage contenant de préférence au moins vingt étages théoriques de lavage, dans laquelle il est mis en contact, à contre-courant, avec un solvant injecté dans la partie supérieure de la colonne 5 par un conduit 6, cette mise en contact étant effectuée à une température comprise, par exemple, entre 0°C et -45°C. En tête de la colonne 5 on recueille, par un conduit 7, un gaz traité consistant principalement en méthane et pauvre en $H_2S$ tandis qu'en fond de ladite colonne on soutire, par un conduit 8, une phase liquide formée du solvant enrichi en $H_2S$ et autres composés absorbés, ladite phase liquide étant réunie, dans un conduit 9a, aux condensats arrivant du séparateur 2a par le conduit 4 pour constituer une phase liquide appelée solvant riche. On choisit une température finale dans l'intervalle 0°C à -30°C pour l'étape de réfrigération dans la zone 2 et l'on réalise la mise en contact du mélange gazeux prétraité avec le solvant dans la colonne 5 à une température suffisamment basse dans l'intervalle 0°C à -45°C et avec un rapport des débits massiques de mélange gazeux à traiter et de solvant de telle sorte que d'une part le gaz traité recueilli, par le conduit 7, en tête de la colonne 5 ait une pression partielle en $H_2S$ inférieure à 65 Pa et, si le mélange gazeux à traiter renferme COS et/ou des mercaptans en plus de l'$H_2S$, une pression partielle globale en composés soufrés inférieure à 260 Pa et que d'autre part le solvant riche, passant dans le conduit 9a, renferme au moins 80% molaire des hydrocarbures en $C_3$ et plus présents dans le mélange gazeux à traiter.

Le solvant riche circulant dans le conduit 9a est introduit dans la partie supérieure d'une colonne 9 de déméthanisation, consistant en une colonne de distillation à rebouillage et dans laquelle le solvant riche est fractionné en une phase gazeuse riche en méthane, que l'on évacue en tête de la colonne 9 par un conduit 10, et en une phase liquide appauvrie en méthane, appelée solvant riche déméthanisé, qui est soutirée en fond de la colonne 9 par un conduit 11. Le solvant riche déméthanisé est amené dans une zone 12 de réfrigération, dans laquelle il est refroidi à une température comprise entre, par exemple, -25°C et -80°C et suffisamment inférieure à la température règnant dans la zone 5 de lavage pour provoquer une démixtion dudit solvant riche déméthanisé, le dit solvant se séparant alors dans un séparateur 12a,

d'une part en une fraction liquide inférieure soutirée du séparateur par un conduit 14, ladite fraction inférieure, appelée solvant riche purifié, renfermant les composés acides $H_2S$ et le cas échéant $CO_2$, COS et mercaptans en solution dans le solvant et contenant moins de 5% molaire d'hydrocarbures, exprimés en équivalent méthane, par rapport aux composés acides et d'autre part en une fraction liquide supérieure évacuée du séparateur 12a par un conduit 13, ladite fraction liquide supérieure, appelée coupe primaire d'hydrocarbures lourds, étant formée du reste des hydrocarbures qui étaient présents dans le solvant riche déméthanisé et comprend au moins 80 % molaire des hydrocarbures en $C_3$ et plus contenus dans le mélange gazeux à traiter.

Le solvant riche purifié subit alors une détente jusqu'à une pression supérieure à 100 KPa et de préférence comprise entre 150 KPa et 300 KPa par passage à travers une vanne 15 de détente, puis le solvant détendu traverse un réchauffeur 16, dans lequel on lui fait subir une vaporisation partielle, le fluide résultant de cette vaporisation étant amené dans un séparateur 16a, duquel on évacue en tête, par un conduit 17, une fraction de gaz acide et on soutire en fond, par un conduit 18, un liquide appelé solvant semi-régénéré. La détente du solvant riche purifié suivie de la vaporisation partielle du solvant détendu, qui assurent une régénération partielle du solvant riche purifié, sont conduites de manière à obtenir un solvant semi-régénéré dont la température est supérieure ou égale à 0°C et la pression est inférieure à la pression du solvant détendu et au moins égale à 100 KPa. Le solvant semi-régénéré passant dans le conduit 18 est introduit dans la partie supérieure d'une colonne à distiller à rebouillage, dans laquelle il subit une régénération totale. Dans ladite colonne 20, le solvant semi-régénéré est séparé, par distillation, en une fraction de gaz acide, évacuée en tête de la colonne par un conduit 19, et en un solvant régénéré, soutiré en fond de ladite colonne par un conduit 21, ledit solvant régénéré étant recyclé par le conduit 6 dans la colonne 5 de lavage après refroidissement à une température appropriée dans une zone 22 de réfrigération.

Les fractions de gaz acide évacuées d'une part en tête du séparateur 16a, par le conduit 17, et d'autre part de la colonne 20, par le conduit 19, sont mélangées pour former le courant de gaz acide, dont la teneur en hydrocarbures, exprimée en équivalent méthane, est inférieure à 5 % molaire par rapport aux composés acides.

Le gaz traité, recueilli par le conduit 7 à la température régnant dans la zone de lavage 5, peut être livré au réseau de distribution après réchauffage ou subir au préalable, le cas échéant, un ou plusieurs traitements additionnels tels que lavage par un solvant approprié pour récupérer le $CO_2$ qu'il peut encore contenir ou traitement dans un turboexpandeur pour récupérer les hydrocarbures en $C_2$ à $C_3$, qui sont alors réunis, après séparation du gaz traité, à la coupe d'hydrocarbures lourds évacuée du séparateur 12a par le conduit 13.

En se référant à la figure 2, le mélange gazeux à traiter arrivant par un conduit 1 subit une réfrigération, dans une zone 2 réfrigération, jusqu'à une température comprise, par exemple, entre 0°C et -30°C et se sépare, dans un séparateur 2a, d'une part en une phase liquide appelée condensats et contenant principalement des hydrocarbures en $C_3$ et plus et de l'$H_2S$, ladite phase liquide étant soutirée en fond du séparateur par un conduit 4, et d'autre part en une phase gazeuse, dite mélange gazeux prétraité, sortant en tête du séparateur par un conduit 3. Ledit mélange gazeux prétraité est introduit dans la partie inférieure d'une colonne 5 de lavage contenant de préférence au moins vingt étages théoriques de lavage, dans laquelle il est mis en contact, à contre-courant, avec un solvant froid injecté dans la partie supérieure de la colonne 5 par un conduit 6, cette mise en contact étant effectuée à une température comprise, par exemple, entre 0°C et -45°C. En tête de la colonne 5 on évacue, par un conduit 64, un gaz traité consistant principalement en méthane et pauvre en $H_2S$ tandis qu'en fond de ladite colonne on soutire, par un conduit 8, une phase liquide formée du solvant enrichi en $H_2S$ et autres composés absorbés. Le gaz traité circulant dans le conduit 64 est réfrigéré d'au moins 15°C dans une zone 55 de réfrigération et passe alors dans un séparateur 56, dans lequel il se sépare, du fait de sa réfrigération additionnelle dans la zone 55, en une phase gazeuse que l'on recueille en tête du séparateur 56 par un conduit 7 et en une coupe liquide que l'on soutire en fond dudit séparateur par un conduit 57.

Le solvant enrichi en $H_2S$ et autres composés acides, que l'on soutire par le conduit 8 de la colonne 5, et les condensats issus du séparateur 2a par le conduit 4 sont mélangés pour constituer une phase liquide, appelée solvant riche, à laquelle on ajoute la coupe liquide issue du séparateur 56 par le conduit 57, l'ensemble s'écoulant dans le conduit 9a. On choisit une température finale dans l'intervalle 0°C à -30°C pour l'étape de réfrigération dans la zone 2 et l'on réalise la mise en contact du mélange gazeux prétraité avec le solvant dans la colonne 5 à une température suffisamment basse dans l'intervalle 0°C à -45°C et avec un rapport des débits massiques de mélange gazeux à traiter et de solvant de telle sorte que d'une part le gaz traité recueilli, par le conduit 64, en tête de la colonne 5 ait une pression partielle en $H_2S$ inférieure à 65 Pa et, si le mélange gazeux à traiter renferme COS et/ou des mercaptans en plus de l'$H_2S$, une pression partielle globale en composés soufrés inférieure à 260 Pa et que d'autre part le solvant riche, passant dans le conduit 9a, renferme au moins 80 % molaire des hydrocarbures en $C_3$ et plus présents dans le mélange gazeux à traiter. La colonne 5 comporte deux réfrigérants 61 parcourus par le milieu fluide, à savoir mélange de solvant et de gaz à traiter, contenu dans ladite colonne et permettant audit milieu d'échanger indirectement des calories avec un fluide réfrigérant de manière à limiter l'échauffement dudit milieu du fait de l'absorption et à obtenir un profil uniforme de température à ± 5°C près dans la colonne.

Le solvant riche circulant dans le conduit 9a est introduit dans la partie supérieure d'un ballon de détente 29 de manière à réaliser une détente dudit solvant riche à une pression intermédiaire propre à libérer une fraction importante du méthane dissous dans le solvant riche et à produire un premier gaz riche en méthane, que l'on évacue en tête du ballon 29 par un conduit 34, et un solvant riche prédéméthanisé, que l'on soutire en fond du ballon 29 par un conduit 35.

Ledit solvant riche prédéméthanisé est soumis à une seconde détente suivie d'une distillation dans une colonne de distillation 9 comportant un rebouillage 30 réalisé, dans deux rebouilleurs 30a et 30b placés en série, au moyen d'un fluide de rebouillage 31 consistant soit en au moins une partie du mélange gazeux à traiter ou encore en une partie du solvant régénéré, de manière à produire un second gaz riche en méthane, que l'on évacue en tête de la colonne 9 par un conduit 32, et une phase liquide appauvrie en méthane, appelée solvant riche déméthanisé, qui est soutirée en fond de la colonne 9 par un conduit 11. Le second gaz riche en méthane circulant dans le conduit 32 est amené à passer dans un compresseur 33 d'où il sort, par un conduit 36, à une pression sensiblement égale à celle du premier gaz riche en méthane passant dans le conduit 34, puis ces deux gaz riches en méthane sont mélangés dans le conduit 10 et la phase gazeuse résultant de ce mélange est recyclée, par l'intermédiaire d'un compresseur 23, dans le mélange gazeux à traiter arrivant par le conduit 1.

Le solvant riche déméthanisé est amené dans une zone 12 de réfrigération, dans laquelle il est refroidi à une température comprise entre, par exemple, -25°C et -80°C et suffisamment inférieure à la température règnant dans la zone 5 de lavage pour provoquer une démixtion dudit solvant riche déméthanisé en deux fractions, qui se séparent, dans un séparateur 12a, en une fraction liquide inférieure soutirée du séparateur par un conduit 38, ladite fraction étant appelée solvant prépurifié et consistant en une solution des composés acides $H_2S$ et le cas échéant $CO_2$, COS et mercaptans dans le solvant, ladite solution renfermant également une petite quantité d'hydrocarbures, et en une fraction liquide supérieure, appelée coupe primaire d'hydrocarbures lourds, qui est évacuée du séparateur 12a par un conduit 37. Le solvant prépurifié est alors introduit dans la partie supérieure d'une zone primaire 39 d'extraction liquide-liquide, qui comporte au moins quatre étages théoriques d'extraction et dans laquelle ledit solvant est mis en contact, à contre-courant, avec un agent d'extraction amené dans la partie inférieure de la zone 39 par un conduit 40.

L'agent d'extraction consiste principalement en hydrocarbures dont la masse moléculaire est supérieure à celle du n-pentane. L'opération d'extraction conduit à séparer une coupe secondaire liquide d'hydrocarbures, qui est évacuée de la zone 39 d'extraction par un conduit 62, d'un solvant riche purifié, qui est soutiré de la zone d'extraction 39 par un conduit 14 et renferme en solution les composés acides $H_2S$ et le cas échéant $CO_2$, COS et mercaptans et moins de 5 % molaire d'hydrocarbures, exprimés en équivalent méthane, par rapport aux composés acides. La coupe secondaire d'hydrocarbures passant dans le conduit 62 est réunie à la coupe primaire d'hydrocarbures s'écoulant par le conduit 37 et le mélange ainsi réalisé est amené, à travers un réchauffeur 41, dans une colonne à distiller 42, dans laquelle ledit mélange est fractionné de manière à évacuer en tête de la colonne 42, par un conduit 13, une coupe d'hydrocarbures lourds renfermant au moins 80 % molaire des hydrocarbures en $C_3$ et plus présents dans le mélange gazeux à traiter et à récupérer en fond de ladite colonne, par un conduit 43, l'agent d'extraction, dont une partie principale est recyclée vers la zone 39 d'extraction, par le conduit 40, après refroidissement dans une zone 44 de réfrigération à une température sensiblement égale à celle régnant dans la zone 12 de démixtion. La partie restante de l'agent d'extraction est évacuée par un conduit 45.

Le solvant riche purifié s'écoulant dans le conduit 14 subit alors une détente jusqu'à une pression supérieure à 100 KPa et de préférence comprise entre 150 KPa et 300 KPa par passage à travers une vanne 15 de détente, puis le solvant détendu traverse un réchauffeur primaire 46, dans lequel on lui fait subir une vaporisation partielle primaire et l'on amène le fluide résultant de cette vaporisation primaire dans un séparateur 46a, duquel on évacue en tête, par un conduit 47, une fraction primaire de gaz acide et on soutire en fond, par un conduit 50, une phase liquide primaire dont la température est inférieure à 0°C. Ladite phase liquide primaire passe alors dans un réchauffeur secondaire 16, dans lequel elle est soumise à une vaporisation partielle secondaire et l'on amène le fluide résultant de cette vaporisation secondaire dans un séparateur 16a, duquel on évacue en tête, par un conduit 17, une seconde fraction de gaz acide et on soutire en fond, par un conduit 18, une phase liquide secondaire, appelée solvant semi-régénéré, dont la température est égale ou supérieure à 0°C et la pression est inférieure à la pression du solvant détendu et au moins égale à 100 KPa. Cette succession d'opérations constitue l'étape de régénération partielle du solvant riche purifié. Le solvant semi-régénéré est ensuite soumis à une étape de régénération totale. Pour ce faire, le solvant semi-régénéré, qui sort du séparateur 16a par le conduit 18, est divisé en deux courants, dont l'un est introduit, par un conduit 51, sur le plateau de tête d'une colonne à distiller 20 à rebouillage comportant une pluralité d'étages théoriques et l'autre est injecté dans ladite colonne en un point intermédiaire de cette dernière par un conduit 52 après avoir été réchauffé dans un échangeur indirect de chaleur 52a. Dans la colonne 20, le solvant semi-régénéré est séparé, par distillation, en une fraction de gaz acide, évacuée en tête de la colonne par un conduit 19 et en un solvant régénéré, soutiré en fond de ladite colonne par un conduit 21, ledit solvant régénéré étant utilisé, dans l'échangeur de chaleur 52a, pour réchauffer le courant de solvant semi-régénéré injecté dans la colonne 20 par le conduit 52, avant d'être recyclé, par un conduit 6, dans la colonne 5 de lavage à travers une zone 22 de réfrigération, qui assure un refroidissement du solvant régénéré à une température appropriée pour son injection dans la colonne 5.

La fraction primaire de gaz acide, évacuée du séparateur 46a par le conduit 47, est portée à une température supérieure ou égale à 0°C dans un réchauffeur 49 puis s'écoule dans un conduit 48 pour être mélangée avec la fraction secondaire de gaz acide, évacuée du séparateur 16a par le conduit 17, et avec la fraction de gaz acide évacuée de la colonne de régénération totale 20 par le conduit 19, le mélange de ces trois fractions de gaz acide constituant le courant de gaz acide produit par le procédé et dont la teneur en hydrocarbures, exprimée en équivalent méthane, est inférieure à 5 % molaire par rapport aux composés acides.

Le gaz traité, recueilli par le conduit 7 à la température régnant dans le séparateur 56, peut être délivré au réseau de distribution après réchauffage ou subir au préalable, si désiré, un ou plusieurs traitements additionnels comme indiqué précédemment pour le mode de réalisation se référant à la figure 1.

Le mode de réalisation du procédé suivant l'invention, qui est illustré par la figure 3, comporte toutes les étapes du mode de réalisation se référant à la figure 2 auxquelles s'ajoutent une étape de séchage et débenzolage réalisée sur le mélange gazeux à traiter avant toute autre opération et une étape de purification additionnelle par extraction réalisée sur le gaz traité réfrigéré recueilli en tête du séparateur 56.

Le mélange gazeux à traiter arrivant par le conduit 1 est introduit dans la partie inférieure d'une colonne 25 de rebouillage, dans laquelle ledit mélange gazeux est mis en contact, à contre-courant, avec du solvant prélevé, par un conduit 54 débouchant dans la partie supérieure de la colonne 25, sur le solvant régénéré en aval de l'échangeur de chaleur 52a et avant passage dudit solvant dans la zone 22 de réfrigération, de manière à produire d'une part un mélange gazeux sec, évacué de la colonne 25 par un conduit 26 et dont la teneur en hydrocarbures aromatiques, notamment benzène, est inférieure à 0,1 % en poids, et d'autre part une coupe hydrocarbonée aromatique, soutirée de la colonne 25 par un conduit 27 et renfermant la plus grande partie des hydrocarbures aromatiques contenus dans le mélange gazeux à traiter et un liquide soutiré de la colonne 25 par un conduit 28 et consistant en solvant renfermant l'eau absorbée.

Le mélange gazeux sec circulant dans le conduit 26 est ensuite soumis au même traitement que le mélange gazeux arrivant par le conduit 1 dans le mode de réalisation de la figure 2 avec toutefois d'une part utilisation dans la colonne 5 de lavage de trois réfrigérants 61 pour le contrôle de la température dans ladite colonne et d'autre part recyclage de la phase gazeuse riche en méthane, qui est issue de la déméthanisation et s'écoule dans le conduit 10, dans le mélange gazeux à traiter avant son introduction dans la colonne 25 de séchage et débenzolage.

Le gaz traité réfrigéré issu du séparateur 56 est amené, par un conduit 58, dans la partie inférieure d'une zone complémentaire d'extraction 60, dans laquelle il est mis en contact, à contre-courant, avec un agent d'extraction introduit dans la partie supérieure de ladite colonne, de manière à produire un gaz traité de pureté améliorée, évacué en tête de la zone 60 par un conduit 7, et une fraction liquide que l'on soutire, par un conduit 61, en fond de ladite zone et que l'on réunit au mélange des coupes primaire et secondaire d'hydrocarbures amené au réchauffeur 41. En tant qu'agent d'extraction injecté dans la zone d'extraction 60, on utilise la partie de l'agent d'extraction soutiré de la colonne de distillation 42, qui n'est pas utilisée pour alimenter la zone primaire d'extraction 39, après l'avoir réfrigérée dans une zone 59 de réfrigération.

Le gaz traité, recueilli par le conduit 7 à la température régnant dans la zone 60 d'extraction, peut être délivré au réseau de distribution après réchauffage ou subir au préalable, si désiré, un ou plusieurs traitements additionnels comme indiqué pour les modes de réalisation se référant aux figures 1 et 2.

En se référant à la figure 4, le mélange gazeux à traiter arrivant par un conduit 1 est introduit dans la partie inférieure d'une tour 70 dans laquelle il est refroidi jusqu'à une température comprise, par exemple, entre 0°C et -30°C et se sépare d'une part en une phase liquide appelée condensats et contenant principalement des hydrocarbures en $C_3$ et plus et de l'$H_2S$, ladite phase liquide étant soutirée en fond de la tour 70 par un conduit 71, et d'autre part en une phase gazeuse, dite mélange gazeux prétraité, sortant en tête de la tour 70 par un conduit 3. Ledit mélange gazeux prétraité est introduit dans la partie inférieure d'une colonne 5 de lavage contenant de préférence au moins vingt étages théoriques de lavage, dans laquelle il est mis en contact, à contre-courant, avec un solvant froid injecté dans la partie supérieure de ladite colonne 5 par un conduit 6, cette mise en contact étant effectuée à une température comprise, par exemple, entre 0°C et -45°C. En tête de la colonne 5 on évacue, par un conduit 64, un gaz traité consistant principalement en méthane et pauvre en $H_2S$ tandis qu'en fond de ladite colonne on soutire, par un conduit 8, une phase liquide formée du solvant enrichi en $H_2S$ et autres composés absorbés.

La phase liquide soutirée de la tour 70 par le conduit 71 est distillée dans une colonne 72 de manière à produire des condensats, soutirés en fond de ladite colonne par un conduit 73, et une phase gazeuse, qui est évacuée de la colonne 72, par un conduit 74, puis réfrigérée dans une zone 75 de réfrigération avant d'être mélangée au solvant enrichi circulant dans le conduit 8 pour former une phase liquide appelée solvant riche, qui s'écoule dans le conduit 9a. On choisit une température dans l'intervalle 0°C à -30°C pour l'étape de refroidissement dans la tour 70 et l'on réalise la mise en contact du mélange gazeux prétraité avec le solvant dans la colonne 5 à une température suffisamment basse dans l'intervalle 0°C à -45°C et avec un rapport des débits massiques de mélange gazeux à traiter et de solvant de telle sorte que d'une part le gaz traité recueilli par le conduit 64, en tête de la colonne 5, ait une pression patielle en

$H_2S$ inférieure à 65 Pa et, si le mélange gazeux à traiter renferme COS et/ou des mercaptans en plus de l'$H_2S$, une presseion partielle globale en composés soufrés inférieure à 260 Pa et que d'autre part le solvant riche, passant dans le conduit 9a, renferme au moins 80 % molaire des hydrocarbures en $C_3$ et plus présents dans le mélange gazeux à traiter. La colonne 5 comporte deux réfrigérants, 61a et 61b parcourus par le milieu fluide, à savoir mélange de solvant et de gaz à traiter, contenu dans ladite colonne et permettant audit milieu d'échanger indirectement des calories avec un fluide réfrigérant de manière à limiter l'échauffement dudit milieu du fait de l'absorption et à obtenir un profil uniforme de température dans la colonne.

Le solvant riche circulant dans le conduit 9a est introduit dans la partie supérieure d'un ballon de détente 29 de manière à réaliser une détente dudit solvant riche à une pression intermédiaire propre à libérer une fraction importante du méthane dissous dans le solvant riche et à produire un premier gaz riche en méthane, que l'on évacue en tête du ballon 29, par un conduit 34, et un solvant riche prédéméthanisé, que l'on soutire en fond du ballon 29 par un conduit 35. Ledit solvant riche prédéméthanisé est soumis à une seconde détente suivie d'une distillation dans une colonne de distillation 9 comportant un système de rebouillage 30, de manière à produire un second gaz riche en méthane, que l'on évacue en tête de la colonne 9 par un conduit 32 et une phase liquide appauvrie en méthane, appelée solvant riche déméthanisé, qui est soutirée en fond de colonne 9 par un conduit 11. Le second gaz riche en méthane circulant dans le conduit 32 est amené à passer dans un compresseur 33 d'où il sort, par un conduit 36, à une pression sensiblement égale à celle du premier gaz riche en méthane passant dans le conduit 34, puis ces deux gaz riches en méthane sont mélangés dans le conduit 10 et la phase gazeuse résultant de ce mélange est amenée, par l'intermédiaire d'un compresseur 23, dans une zone de réfrigération 76, dans laquelle ladite phase gazeuse est amenée à une température sensiblement égale à celle du mélange gazeux prétraité circulant dans le conduit 3. La phase gazeuse issue de la zone de réfrigération 76 est partiellement liquéfiée. Ladite phase partiellement liquéfiée est introduite dans un séparateur 77, dans lequel elle se sépare en une fraction liquide, qui est soutirée du séparateur par un conduit 79 et mélangée au solvant enrichi passant dans le conduit 8 et en une fraction gazeuse, qui est évacuée du séparateur 77 par un conduit 78 et incorporée au mélange gazeux prétraité passant dans le conduit 3.

Le solvant riche déméthanisé est amené dans une zone 12 de réfrigération, dans laquelle il est refroidi à une température comprise entre, par exemple, -25°C et - 80°C et suffisamment inférieure à la température régnant dans la zone 5 de lavage pour provoquer une démixtion dudit solvant riche déméthanisé en deux fractions, qui se séparent, dans un séparateur 12a, en une fraction liquide inférieure soutirée du séparateur par un conduit 14, ladite fraction étant appelée solvant purifié et consistant en une solution des composés acides $H_2S$ et le cas échéant $CO_2$, COS et mercaptans dans le solvant, ladite solution renfermant également une très faible quantité d'hydrocarbures, et en une fraction liquide supérieure, appelée coupe primaire d'hydrocarbures lourds, qui est évacuée du séparateur 12a par un conduit 37.

Le solvant riche purifié s'écoulant dans le conduit 14 est alors soumis à un traitement de régénération comme indiqué en référence à la figure 2 pour produire d'une part le solvant régénéré, qui est recyclé, par un conduit 6, dans la colonne 5 de lavage à travers une zone de réfrigération 22 assurant un refroidissement du solvant régénéré à une température appropriée pour son injection dans la colonne 5 et d'autre part le courant de gaz acide 24 fourni par le procédé et renfermant une teneur en hydrocarbures, exprimée en équivalent méthane, inférieure à 5 % molaire par rapport aux composés acides.

Pour compléter la description précédente, on donne ci-après, à titre non limitatif, quatre exemples de mise en oeuvre du procédé suivant l'invention.

EXEMPLE 1 :

En faisant appel à une installation analogue à celle schématisée sur la figure 2 du dessin annexé et fonctionnant comme décrit précédemment, on traitait un mélange gazeux ayant la composition molaire suivante :

. Méthane : 69 %
. Ethane : 3 %
. Propane : 1 %
. Butane : 1 %
. Hexane : 1 %
. $H_2S$ : 15 %
. $CO_2$ : 10 %

Le mélange gazeux à traiter, arrivant par le conduit 1 avec un débit de 20 000 Kmoles/heure, une température de 30°C et une pression de 5 MPa, était mélangé à la phase gazeuse riche en méthane, issue de la déméthanisation du solvant riche et délivrée par le compresseur 23, et le mélange gazeux obtenu était refroidi jusqu'à -10°C dans la zone 2 de réfrigération. Cette réfrigération produisait 872 Kmoles/h de condensats, évacués du séparateur 2a par le conduit 4, et 21 039 Kmoles/h de gaz prétraité, évacué du séparateur 2a par le conduit 3, dont la composition molaire était la suivante :

. Méthane : 70,31 %
. Ethane : 3,20 %
. Propane : 0,85 %

. Butane : 0,51 %
. Hexane : 0,11 %
. $H_2S$ : 13,89
. $CO_2$ : 11,13%

Le mélange gazeux prétraité était mis en contact avec 14 000 Kmoles/h de solvant consistant en méthanol pur, ladite mise en contact étant réalisée dans une colonne 5 de lavage comportant 28 étages théoriques et pourvue de deux réfrigérants 61 latéraux situés l'un au niveau du premier plateau et l'autre au niveau du 28ème plateau et dont les puissances sont respectivement de 40 et 37,8 G Joules/heure, ce qui permet de limiter l'échauffement dû à l'absorption et d'obtenir un profil uniforme de températuré à ± 5°C près dans la colonne 5, ladite colonne opérant à une température de - 10°C.

En tête de la colonne 5, on évacuait un gaz traité ayant une pression de 4959 KPa et une température de -10°C et présentant la composition suivante en pourcentage molaire :
. Méthane : 91,71
. Ethane : 2,99
. Propane : 0,15
. Butane : 0
. Hexane : 0
. $H_2S$ : 10 p.p.m. (en volume)
. $CO_2$ : 5,06
. Méthanol : 0,09

La pression partielle en $H_2S$ dudit mélange gazeux traité était égale à 50 Pa.

Le mélange gazeux traité issu de la colonne 5, par le conduit 64, était réfrigéré jusqu'à - 45°C, dans la zone 55 de réfrigération, de manière à condenser, dans le séparateur 56, plus de 90 % du méthanol entraîné par le mélange gazeux hors de la colonne 5 de lavage. La coupe liquide, condensée dans le séparateur 56 et soutirée dudit séparateur, par le conduit 57, avec un débit de 15 Kmoles/h, renfermait principalement du méthanol et du $CO_2$.

Les condensats, soutirés du séparateur 2a par le conduit 4, le solvant enrichi en composés acides, soutiré de la colonne 5 par le conduit 8, et la coupe liquide, soutirée du séparateur 56 par le conduit 57, étaient mélangés pour constituer le solvant riche soumis à la déméthanisation, ledit solvant riche ayant un débit égal à 21 270 Kmoles/h et présentant la composition suivante en pourcentage molaire :
. Méthane : 5,96
. Ethane : 1,34
. Propane : 0,97
. Butane : 0,99
. Hexane : 0,95
. Méthanol : 65,82
. $H_2S$ : 15,91
. $CO_2$ : 8,05

La déméthanisation du solvant riche comportait tout d'abord une première détente dudit solvant à une pression de 2420 KPa dans le ballon 29 permettant de séparer 61 % du méthane dissous avec production de 1149 Kmoles/h d'un gaz renfermant 68 % molaire de méthane, évacué en tête du ballon 29 par le conduit 34, et un solvant riche prédéméthanisé soutiré dudit ballon par le conduit 35, puis une seconde détente à 1050 KPa du solvant riche prédéméthanisé suivie d'une distillation dans la colonne 9 comportant 11 plateaux théoriques et dont le rebouillage est effectué par le mélange gazeux à traiter de manière à produire 1148 Kmoles/h d'un second gaz riche en méthane, évacué en tête de la colonne 9 par le conduit 32, et le solvant riche déméthanisé disponible à 13°C et 1070 KPa et dont la composition, en pourcentage molaire est la suivante:
. Méthane : 0,02
. Ethane : 0,79
. Propane : 0,94
. Butane : 1,05
. Hexane : 1,05
. Méthanol : 73,78
. $H_2S$ : 15,81
. $CO_2$ : 6,54

Le second gaz riche en méthane était comprimé, dans le compresseur 33, jusqu'à 2420 KPa, puis mélangé au premier gaz riche en méthane, pour constituer la phase gazeuse riche en méthane recyclée dans le mélange gazeux à traiter par l'intermédiaire du compresseur 23.

Le solvant riche déméthanisé était refroidi, dans la zone 12 de réfrigération, jusqu'à une température de -75°C pour provoquer sa démixtion en deux fractions, qui se séparaient, dans le séparateur 12a, en une coupe primaire d'hydrocarbures lourds, évacuée par le conduit 37, et en un solvant prépurifié, soutiré par le conduit 38.

Ledit solvant prépurifié avait la composition suivante en pourcentage molaire :
. Méthane : 0,02
. Ethane : 0,55

. Propane : 0,58
. Butane : 0,45
. Hexane : 0,09
. Méthanol : 76,20
. $H_2S$ : 15,54
. $CO_2$ : 6,58

Le solvant prépurifié, circulant dans le conduit 38, était mis en contact, dans le dispositif d'extraction liquide-liquide 39 comportant 5 étages théoriques et opérant à -75°C, avec 600 kmoles/h d'un agent d'extraction composé principalement d'hexane, le résultat de cette extraction étant la production d'une coupe secondaire liquide d'hydrocarbures, évacuée du dispositif 39 par le conduit 62, et d'un solvant purifié soutiré par le conduit 14.

Ledit solvant purifié présentait la composition suivante en pourcentage molaire :
. Méthane : 0,01
. Ethane : 0,08
. Propane : 0,04
. Butane : 0,01
. Hexane : 0,12
. Méthanol : 79,08
. $H_2S$ : 15,54
. $CO_2$ : 6,40

La coupe secondaire d'hydrocarbures, passant dans le conduit 62, était réunie à la coupe primaire d'hydrocarbures, s'écoulant par le conduit 37, et le mélange ainsi obtenu était réchauffé, dans le réchauffeur 41, puis fractionné, par distillation dans la colonne 42, en une coupe d'hydrocarbures lourds, évacuée par le conduit 13 et renfermant 96 % molaire des hydrocarbures en $C_3$ et plus contenus dans le mélange gazeux à traiter arrivant par le conduit 1, et en une fraction liquide consistant en l'agent d'extraction et soutirée par le conduit 43. Une partie principale dudit agent d'extraction était refroidie jusqu'à -75°C, par passage dans la zone de réfrigération 44, puis recyclée vers le dispositif 39 d'extraction par le conduit 40.

Le solvant riche purifié, s'écoulant par le conduit 14, était soumis à un traitement de régénération comportant une étape de régénération partielle, suivie d'une étape de régénération totale. Pour réaliser l'étape de régénération partielle, ledit solvant riche purifié était détendu à 230 KPa en traversant la vanne 15 de détente puis subissait une vaporisation partielle primaire dans le réchauffeur 46 par réchauffage à -25°C avec comme résultat la séparation, dans le séparateur 46a, de 1767 Kmoles/h d'une fraction primaire de gaz acide, recueillie par le conduit 47, d'avec une phase liquide primaire soutirée par le conduit 50. Ladite phase liquide subissait alors une vaporisation partielle secondaire dans le réchauffeur 16 par réchauffage à 7°C sous une pression de 190 KPa avec comme résultat la séparation, dans le séparateur 16a, de 1133 Kmoles/h d'une fraction secondaire de gaz acide, évacuée par le conduit 17, d'avec un solvant semi-régénéré soutiré par le conduit 18 et ayant une température égale à 7°C.

La régénération totale du solvant semi-régénéré était effectuée en pompant tout d'abord ledit solvant jusqu'à 450 KPa puis en le divisant en deux courants. L'un des courants, représentant un débit de 5000 Kmoles/h, était envoyé, par le conduit 51, sur le plateau de tête de la colonne de distillation 20, qui opère sous une pression de 350 KPa, tandis que l'autre courant était réchauffé, dans l'échangeur 52a, jusqu'à 88°C par échange indirect de chaleur avec le solvant régénéré soutiré de la colonne 20 par le conduit 21, puis introduit dans la colonne 20 au niveau du septième étage théorique. La distillation du solvant semi-régénéré dans la colonne 20 produisait 799 Kmoles/h d'une fraction de gaz acide recueillie par le conduit 19 et du solvant régénéré soutiré par le conduit 21. Ledit solvant régénéré quittait l'échangeur de chaleur 52a avec une température de 30°C et était recyclé dans la colonne 5, par le conduit 6, après refroidissement à -10°C dans la zone de réfrigération 22.

La fraction primaire de gaz acide s'écoulant dans le conduit 47 était portée à une température de 7°C dans le réchauffeur 49, puis elle était réunie à la fraction secondaire de gaz acide passant dans le conduit 17 et à la fraction de gaz acide évacuée de la colonne 20 par le conduit 19 pour former un courant de gaz acide ayant une pression de 190 KPa et un débit de 3699 Kmoles/h, ledit courant de gaz acide ayant en outre une teneur en hydrocarbures, exprimée en équivalent méthane, égale à 1,9 % molaire et une teneur en méthanol de 1,14 % en poids. Le rapport molaire $H_2S$ : $CO_2$ dans ce courant de gaz acide était égal à 2,22 alors que la valeur de ce même rapport dans le mélange gazeux à traiter était seulement de 1,5.

Le courant de gaz acide s'écoulant par le conduit 24 était ensuite soumis à un lavage conventionnel à l'eau, avant d'être dirigé vers une unité à soufre CLAUS.

EXEMPLES 2 A 4

En faisant appel à une installation analogue à celle schématisée sur la figure 4 du dessin annexé et fonctionnant comme décrit précédemment, on traitait un mélange gazeux ayant une composition, un débit, une température et une pression identiques ceux à du mélange gazeux traité dans l'exemple 1.

Le solvant utilisé était différent d'un exemple à l'autre et consistait en carbonate de propylène pur

(exemple 2), en diméthylformamide pur (exemple 3) et en γ-butyrolactone pure (exemple 4).

Le mode opératoire suivant était utilisé dans les trois exemples.

Le mélange gazeux à traiter était refroidi jusqu'à - 24°C dans la tour 70, cette réfrigération produisant 1226 kmoles/h de condensats soutirés par le conduit 71 et 18774 kmoles/h de mélange gazeux prétraité sortant de la tour 70 par le conduit 3.

Ledit gaz prétraité avait la composition suivante, en pourcentage molaire,dans les trois exemples.

. méthane : 72,35
. éthane : 2,96
. propane : 0,79
. butane : 0,02
. hexane : 0,
. $H_2S$ : 13,67
. $CO_2$ : 10,21

La colonne 5 de lavage, dans laquelle on effectuait la mise en contact du mélange gazeux prétraité avec le solvant, comportait 21 étages théoriques et était équipée de deux réfrigérants latéraux 61a et 61b situés respectivement sur les 11ème et 21ème plateaux, les puissances desdits réfrigérants étant choisies pour limiter l'échauffement dû à l'absorption et obtenir un profil uniforme de température dans ladite colonne à ± 5°C près.

En tête de la colonne 5, on évacuait, par le cndüit 64, un gaz traité ayant une pression de 4950 kPa et une teneur en $H_2S$ correspondant à une pression partielle d'$H_2S$ égale à 5 Pa.

En fond de la colonne 5, on soutirait, par le conduit 8, une phase liquide formée du solvant enrichi en $H_2S$ et autres composés absorbés.

Les condensats formés dans la tour 70 étaient distillés dans la colonne 72 avec production de nouveau condensats soutirés de ladite colonne par le conduit 73 et d'un gaz qui était réfrigéré jusqu'à - 25°C dans la zone 75 de réfrigération, puis mélangé au solvant enrichi passant dans le conduit 8 pour subir la déméthanisation.

La déméthanisation du solvant riche circulant dans le conduit 9a était réalisée en deux étapes. Tout d'abord une détente à une pression de 2450 kPa, dans le ballon de détente 29, permettait de séparer environ 80 % du méthane dissous dans ledit solvant. Cette détente produisait un premier gaz riche en méthane, évacué par le conduit 34, et un solvant riche prédéméthanisé soutiré par le conduit 35. Une seconde détente à 1000 kPa suivie d'une distillation dans la colonne 9 de distillation équipée de 14 plateaux théoriques produisait un second gaz riche en méthane, évacué par le conduit 32, et un solvant riche déméthanisé disponible, dans le conduit 11, à une température de 5°C et sous une pression de 1000 kPa. Le second gaz riche en méthane était comprimé jusquà 2400 kPa dans le compresseur 33, puis mélangé au premier gaz riche en méthane dans le conduit 10, le mélange résultant étant alors comprimé jusqu'à 5500 kPa dans le compresseur 23. Le gaz comprimé riche en méthane issu du compresseur 23 passait dans la zone de réfrigération 76, dans laquelle il était réfrigéré jusqu'à -25°C et partiellement liquéfié, le fluide partiellement liquéfié issu de la zone 76 étant amené dans le séparateur 77. La fraction gazeuse évacuée dudit séparateur, par le conduit 78, était incorporée au mélange gazeux prétraité amené à la colonne 5 de lavage tandis que la phase liquide soutirée du séparateur par le conduit 79 était mélangée au solvant enrichi s'écoulant de la colonne 5 par le conduit 8.

Le solvant riche déméthanisé s'écoulant par le conduit 11 était réfrigéré, dans la zone 12 de réfrigération, pour provoquer sa démixtion en deux fractions, qui se séparaient, dans le séparateur 12a, en une coupe d'hydrocarbures liquide évacuée par le conduit 37 et un solvant purifié soutiré par le conduit 14.

Le solvant purifié était alors soumis à un traitement de régénération comportant une étape de régénération partielle suivie d'une étape de régénération totale comme décrit dans l'exemple 1, pour d'une part fournir, par le conduit 24, un courant de gaz acide à très faible teneur en hydrocarbures et d'autre part produire, par le conduit 21, un solvant régénéré, qui après réfrigération jusqu'à - 25°C, dans la zone 22 de réfrigération, était recyclé à la colonne 5 de lavage par le conduit 6.

Les conditions opératoires et les résultats sépcifiques à chaque exemple sont présentés dans le tableau ci-après.

**TABLEAU**

| EXEMPLE | 2 | 3 | 4 |
|---|---|---|---|
| . Solvant : | carbonate de propylène | diméthyl-formamide | γ-butyrolactone |
| . Débit du solvant arrivant par 6 à la colonne 5 (kmoles/h) : | 5 500 | 2 500 | 5 000 |
| . Puissance en G Joules/h des réfrigérants | | | |
| 61a : | 2 | 8 | 10 |
| 61b : | 29 | 35 | 30 |
| . Gaz traité évacué par le conduit 64 | | | |
| - Température : | - 25 °C | - 15°C | - 25°C |
| - Composition molaire (%) | | | |
| . méthane : | 89,65 | 88,06 | 89,65 |
| . éthane : | 3,45 | 3,33 | 3,45 |
| . propane : | 0.80 | 0.74 | 0.80 |
| . butane : | 0. | 0. | 0. |
| . hexane : | 0. | 0. | 0. |
| . $H_2S$ : | 1 ppm (V/V)* | 1 ppm (V/V) | 1 ppm (V/V) |
| . $CO_2$ : | 6,10 | 7,86 | 6,10 |
| . Solvant : | 0,3 ppm (V/V) | 1 ppm (V/V) | 0,3 ppm (V/V) |

TABLEAU (SUITE)

| EXEMPLE | 2 | 3 | 4 |
| --- | --- | --- | --- |
| . Solvant enrichi soutiré par 8 | | | |
| - Débit (kmoles/h) | 9 410 | 6 160 | 8 910 |
| - Composition molaire (%) | | | |
| . méthane | 2,77 | 4,42 | 2,92 |
| . éthane | 0,46 | 0,86 | 0,48 |
| . propane | 0,29 | 0,60 | 0,31 |
| . butane | 0,02 | 0,04 | 0,02 |
| . solvant | 58,44 | 40,58 | 56,14 |
| : $H_2S$ | 27,76 | 41,67 | 28,79 |
| . $CO_2$ | 10,74 | 11,84 | 11,34 |
| . Déméthanisation | | | |
| - Premier gaz riche en méthane évacué par 34 | | | |
| . Teneur molaire en méthane (%) | 62 | 62 | 62 |
| . Débit (kmoles/h) | 816 | 990 | 800 |
| - Second gaz riche en méthane évacué par 32 | | | |
| . Débit (kmoles/h) | 553 | 881 | 500 |

TABLEAU (SUITE)

| EXEMPLE | 2 | 3 | 4 |
|---|---|---|---|
| - Solvant riche déméthanisé soutiré par 11 | | | |
| - Composition molaire (%) | | | |
| . propane | 0,45 | 0,85 | 0,50 |
| . butane | 0,03 | 0,05 | 0,03 |
| . hexane | 0, | 0, | 0, |
| . solvant | 58,43 | 41,18 | 56,09 |
| . $H_2S$ | 31,04 | 48,28 | 32,78 |
| . $CO_2$ | 10,04 | 9,63 | 10,60 |
| - Réfrigération dans la zone 12 du solvant déméthanisé | | | |
| . Température atteinte | - 45°C | - 50°C | - 45°C |
| - Solvant purifié soutiré par 14 | | | |
| - Composition molaire (%) | | | |
| . propane | 0,10 | 0,17 | 0,10 |
| . butane | 0,02 | 0,05 | 0,02 |
| . hexane | 0, | 0, | 0, |
| . solvant | 60,88 | 42,28 | 58,71 |
| . $H_2S$ | 30,62 | 49,04 | 32,33 |
| . $CO_2$ | 8,37 | 8,45 | 8,84 |

**TABLEAU (SUITE)**

| EXEMPLE | 2 | 3 | 4 |
|---|---|---|---|
| - Régénération du solvant purifié | | | |
| - Solvant régénéré soutiré par 21 | | | |
| . Débit (kmoles/h) | 5 500 | 2 500 | 5 000 |
| . Teneur en $H_2S$ | 1 ppm (V/V) | 1 ppm (V/V) | 1 ppm (V/V) |
| - Gaz acide évacué par 24 | | | |
| - Débit (kmoles/h) | 3 533 | 3 533 | 3 533 |
| - Composition molaire (%) | | | |
| . propane | 0,25 | 0,29 | 0,25 |
| . butane | 0,06 | 0,08 | 0,06 |
| . $H_2S$ | 78,29 | 84,97 | 78,28 |
| . $CO_2$ | 21,40 | 14,65 | 21,41 |

*) ppm (V/V) = partie par million en volume

## Revendications

1- Procédé cryogénique de désulfuration sélective et de dégazolinage simultanés d'un mélange gazeux consistant principalement en méthane et renfermant également $H_2S$, des hydrocarbures en $C_2$ et plus et éventuellement un ou plusieurs composés gazeux choisis parmi les gaz inertes, $H_2O$, $CO_2$, COS et des mercaptans, ce mélange gazeux étant sous une pression absolue supérieure à 0,5 MPa, ledit procédé étant du type dans lequel on met le mélange gazeux en contact dans une zone de lavage (5) avec un solvant (6) consistant en un liquide qui dissout préférentiellement $H_2S$, COS et les mercaptans et seulement une partie du $CO_2$ et qui possède d'une part une température d'ébullition à la pression atmosphérique supérieure à 40°C et d'autre part une viscosité à -40°C inférieure à 0,1 Pa.s et l'on effectue une régénération du solvant renfermant l'$H_2S$ et les autres composés absorbés puis recycle le solvant régénéré vers la zone de lavage et se caractérisant en ce que ladite mise en contact du mélange gazeux avec le solvant est réalisée à une température suffisamment basse et avec un rapport des débits massiques de mélange gazeux à traiter et de solvant et un nombre d'étages théoriques de lavage tels que l'on produise d'une part un gaz traité (7, 64) consistant principalement en méthane et dont la pression partielle en $H_2S$ est inférieure à 65 Pa et, lorsqu'en plus d'$H_2S$ le mélange gazeux à traiter renferme également COS et/ou des mercaptans, la pression partielle globale des composés soufrés est inférieure à 260 Pa et d'autre part une phase liquide appelée solvant riche (8) et composée du solvant enrichi en $H_2S$ et autres composés absorbés et d'une fraction d'hydrocarbures condensés représentant au moins 80 % molaires des hydrocarbures en $C_3$ et plus présents dans le mélange gazeux à traiter, et en ce que l'on soumet le solvant riche à un traitement de déméthanisation au moins partielle (9, 29) pour produire une phase liquide appauvrie en méthane et appelée solvant riche déméthanisé (11) et une phase gazeuse (10) riche en méthane, on refroidit (12) le solvant riche déméthanisé à une température suffisamment inférieure à la température régnant dans la zone de lavage pour provoquer une démixtion dudit solvant déméthanisé en une fraction liquide inférieure appelée solvant riche purifié (14, 38), qui renferme les composés acides $H_2S$ et le cas échéant $CO_2$, COS et mercaptans dissous par le solvant et possède une teneur en hydrocarbures, exprimée en équivalent méthane, inférieure à 5 % molaire de la quantité de composés acides, et en une fraction liquide supérieure (13, 37) appelée coupe primaire d'hydrocarbures lourds et formée du reste des hydrocarbures qui étaient présents dans le solvant riche déméthanisé, on sépare la coupe d'hydrocarbures lourds du solvant riche purifié et l'on soumet ledit solvant riche purifié au traitement de régénération (46, 16, 20) pour produire un courant (24) de gaz acide renfermant la quasi totalité de l'$H_2S$ et autres composés acides éventuels présents dans le solvant riche purifié et contenant, exprimé en équivalent méthane, moins de 5 % molaire d'hydrocarbures par rapport aux composés acides et le solvant régénéré (21), qui est recyclé (6) vers la zone de lavage (5).

2 - Procédé selon la revendication 1, caractérisé en ce que le solvant mis en contact avec le mélange gazeux à à traiter a une viscosité inférieure à 0,05 Pa.s.

3 - Procédé selon la revendication 1 ou 2, caractérisé en ce que le solvant mis en contact avec le mélange gazeux à traiter consiste en un ou plusieurs absorbants organiques liquides, utilisés sous forme anhydre ou en mélange avec de l'eau, le ou lesdits absorbants étant choisis parmi les amides de formules

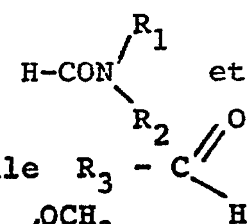 et

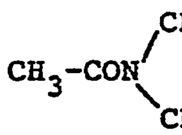, les aldéhydes de formule , les acétals de formules 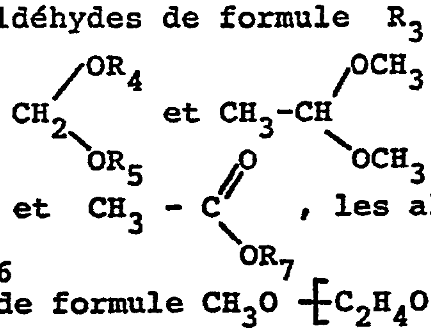 et , les esters de formules 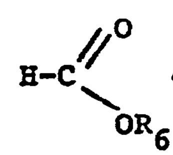 et , les alcanols en $C_1$ à $C_4$, les diéthers de formule $CH_3O - [C_2H_4O]_n - CH_3$, les diéthers alcools de formule $R_9O - C_2H_4-O-C_2H_4-OH$, les lactones de formule 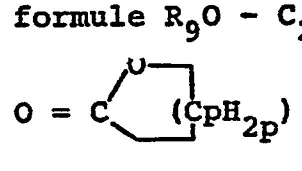 et le carbonate

de propylène, avec dans ces formules $R_1$ et $R_2$, identiques ou différents, désignant un atome d'hydrogène ou un radical alcoyle en $C_1$ ou $C_2$, $R_3$ étant un radical alcoyle en $C_3$ ou $C_4$, $R_4$ et $R_5$, identiques ou différents, représentant un radical alcoyle en $C_1$ à $C_3$, $R_6$ étant un radical alcoyle en $C_2$ à $C_4$ ou un radical

$$\left[ C_2H_4O \right]_n - R_8$$

avec $R_8$ désignant un radical alcoyle en $C_1$ ou $C_2$ et n représentant 1 ou 2, $R_7$ étant un radical alcoyle en $C_1$ ou $C_2$ ou un radical

$$-\left[ C_2H_4O - \right]_n R_8,$$

$R_9$ désignant un radical alcoyle en $C_1$ à $C_4$ et p est un nombre entier allant de 2 à 4.

4 - Procédé selon l'une des revendications 1 à 3, caractérisé en ce que la température de mise en contact du mélange gazeux à traiter avec le solvant, dans la zone de lavage, est comprise entre 0°C et -45°C.

5 - Procédé selon l'une des revendications 1 à 4, caractérisé en ce que la zone de lavage, dans laquelle le mélange gazeux à traiter est mis en contact avec le solvant, consiste en une ou plusieurs colonnes de lavage contenant chacune au moins environ 10 et de préférence au moins 20 étages théoriques de lavage.

6 - Procédé selon la revendication 5, caractérisé en ce que l'on maintient la température dans chacune des colonnes de lavage, de préférence à ± 5°C près, par échange indirect de chaleur (61), effectué en un ou plusieurs points de la colonne considérée, entre le milieu fluide contenu dans cette colonne et un fluide réfrigérant.

7 - Procédé selon d'une des revendications 1 à 6, caractérisé en ce que, préalablement à sa mise en contact avec le solvant, le mélangé gazeux à traiter est soumis à une réfrigération (2) jusqu'à une température comprise entre 0°C et -30°C pour produire d'une part une phase liquide appelée condensats (4) contenant principalement des hydrocarbures en $C_3$ et plus et $H_2S$ et d'autre part une phase gazeuse dite mélange gazeux prétraité (3), puis l'on met ledit mélange gazeux prétraité en contact avec le solvant dans la zone de lavage (5) et l'on réunit le solvant (8) issu de la zone de lavage aux condensats (4) issus de l'étape de réfrigération pour constituer le solvant riche (9a) que l'on soumet au traitement de déméthanisation.

8 - Procédé selon l'une des revendications 1 à 6, caractérisé en ce que, préalablement à sa mise en contact avec le solvant, le mélange gazeux à traiter est soumis à une réfrigération (2) jusqu'à une température comprise entre 0°C et -30°C pour produire d'une part une phase liquide appelée condensats (4) contenant principalement des hydrocarbures en $C_3$ et plus et $H_2S$ et d'autre part une phase gazeuse dite mélange gazeux prétraité (3), puis l'on met ledit mélange gazeux prétraité en contact avec le solvant dans la zone de lavage et l'on soumet chacune des phases liquides constituées par le solvant (8) issu de la zone de lavage (5) et les condensats (4) issus de l'étape de réfrigération (2) à un traitement séparé de déméthanisation, les phases gazeuses produites au cours des traitements de déméthanisation étant éventuellement recyclées, séparément ou en mélange, vers le mélange gazeux (1) à traiter en amont de l'étape de réfrigération (2), et l'on réunit le solvant et les condensats issus des traitements séparés de déméthanisation pour constituer le solvant riche déméthanisé (11).

9 - Procédé selon l'une des revendications 1 à 8, caractérisé en ce que le traitement de déméthanisation appliqué au solvant riche et aux condensats est réalisé en deux étapes, à savoir une première étape dans laquelle le fluide à déméthaniser, à savoir solvant riche ou condensats, est soumis à une première détente (29) à une pression intermédiaire propre à libérer une fraction importante du méthane dissous dans ledit fluide à déméthaniser et à produire un premier gaz riche en méthane (34) et un fluide prédéméthanisé (35) et une seconde étape dans laquelle le fluide prédéméthanisé est soumis à une seconde détente puis à une distillation (9) de manière à produire un second gaz riche en méthane (32) et un fluide déméthanisé (11), le second gaz riche en méthane étant comprimé jusqu'à la pression du premier gaz riche en méthane puis mélangé à ce dernier pour constituer la phase gazeuse (10) riche en méthane.

10 - Procédé selon la revendication 9, caractérisé en ce que, dans la seconde étape du traitement de déméthanisation, la distillation (9) faisant suite à la seconde détente s'effectue par rebouillage réalisé, dans au moins deux zones de rebouillage (30a, 30b) disposées en série, au moyen d'un fluide (31) consistant soit en au moins une partie du mélange gazeux à traiter, prélevée sur ledit mélange gazeux avant sa mise en contact avec le solvant ou avant sa réfrigération, ou encore en une partie du solvant régénéré.

11 - Procédé selon l'une des revendications 1 à 10, caractérisé en ce que la température, inférieure à la température du solvant riche déméthanisé, à laquelle on refroidit (12) ledit solvant pour provoquer sa démixtion est comprise entre -25°C et -80°C.

12 - Procédé selon l'une des revendications 1 à 11, caractérisé en ce que le traitement de régénération du solvant riche purifié comporte une étape de régénération partielle comprenant une détente (15) du

solvant riche purifié jusqu'à une pression supérieure à 100 KPa et de préférence comprise entre 150 KPa et 300 KPa suivie d'au moins une vaporisation (46, 16) partielle dudit solvant détendu de manière à produire d'une part au moins une fraction (47, 17) de gaz acide et d'autre part un solvant semi-régénéré (18) dont la température est au moins égale à 0°C et la pression, inférieure à la pression du solvant détendu, est au moins égale à 100 KPa, puis une étape de régénération totale du solvant semi-régénéré par distillation (20), notamment en faisant appel à un rebouillage, et produisant une fraction de gaz acide (19) et du solvant régénéré (21) ledit solvant régénéré étant recyclé (6), après refroidissement approprié (22), vers la zone de lavage (5) tandis que les différentes fractions de gaz acide (47, 17, 19) sont réunies pour former le courant de gaz acide (24) dont la teneur en hydrocarbures, exprimée en équivalent méthane, est inférieure à 5 % molaire des composés acides.

13 - Procédé selon la revendication 12, caractérisé en ce que, dans l'étape de régénération partielle du solvant riche purifié, la vaporisation partielle du solvant détendu est mise en oeuvre en réalisant tout d'abord une vaporisation partielle primaire (46) dudit solvant de manière à produire une fraction primaire de gaz acide (47) et une phase liquide primaire (50) dont la température est inférieure à 0°C puis en effectuant une vaporisation partielle secondaire (16) de la phase liquide primaire de manière à produire une fraction secondaire de gaz acide (17) et une phase liquide secondaire (18) ayant une température au moins égale à 0°C et une pression, inférieure à la pression du solvant détendu, au moins égale à 100 KPa, ladite phase liquide secondaire constituant le solvant semi-régénéré que l'on traite dans l'étape de régénération totale, la fraction primaire de gaz acide (47) étant réchauffée (49) jusqu'à une température égale ou supérieure à 0°C puis réunie à la fraction secondaire (17) de gaz acide et la fraction de gaz acide (19) résultant de la régénération totale du solvant semi-régénéré pour former le courant de gaz acide (24) dont la teneur en hydrocarbures, exprimée en équivalent méthane, est inférieure à 5 % molaire des composés acides.

14 - Procédé selon la revendication 12 ou 13, caractérisé en ce que, la mise en oeuvre de la régénération totale du solvant semi-régénéré par distillation étant réalisée dans une colonne (20) comportant une pluralité d'étages théoriques avec soutirage (21) du solvant régénéré en fond de colonne, on divise le solvant semi-régénéré en deux courants dont l'un (51) est envoyé sur le plateau de tête de la colonne de distillation et l'autre (52) alimente ladite colonne au niveau d'un étage théorique intermédiaire, après réchauffage à contre-courant (52a) avec le solvant régénéré soutiré de la colonne.

15 - Procédé selon l'une des revendications 1 à 14, caractérisé en ce que le solvant riche purifié (11) est soumis, avant sa régénération, à un traitement complémentaire d'élimination des hydrocarbures, ledit traitement consistant à mettre en contact ledit solvant, dans une zone primaire d'extraction liquide-liquide (39) comportant, de préférence, au moins quatre étages théoriques d'extraction, avec un agent d'extraction principalement constitué d'hydrocarbures dont la masse moléculaire est supérieure à celle du n-pentane, de manière à séparer dudit solvant riche purifié une coupe secondaire liquide (62) d'hydrocarbures, à réunir ladite coupe secondaire (62) à la coupe primaire d'hydrocarbures lourds (37), puis à réchauffer (41) et distiller (42) le mélange ainsi obtenu pour produire la coupe d'hydrocarbures lourds (13) contenant au moins 80 % molaire des hydrocarbures en $C_3$ et plus présents dans le mélange gazeux à traiter et récupérer l'agent d'extraction (43), dont une partie principale est recyclée (40) vers la zone primaire d'extraction liquide-liquide (39), après refroidissement (44) jusqu'à une température sensiblement égale à celle atteinte lors de la démixtion du solvant riche déméthanisé.

16 - Procédé selon l'une des revendications 1 à 15, caractérisé en ce que le gaz traité issu de la zone de lavage est réfrigéré (55) d'au moins 15°C de manière à produire un gaz traité réfrigéré (58) et une coupe liquide (57) qui est soumise au traitement de déméthanisation en mélange avec le solvant riche (9a).

17 - Procédé selon les revendications 15 et 16, caractérisé en ce que l'on soumet le gaz traité réfrigéré, dans une zone complémentaire d'extraction (60), à une extraction additionnelle au moyen de la partie (45) de l'agent d'extraction non utilisée dans la zone d'extraction primaire (39), de manière à produire un gaz traité (7) de pureté améliorée et une fraction liquide (61), que l'on recycle vers la distillation (41, 42) du mélange des coupes primaire et secondaire d'hydrocarbures.

18 - Procédé selon l'une des revendications 1 à 17, caractérisé en ce qu'avant tout traitement le mélange gazeux à traiter est soumis à une opération (25) de séchage et de débenzolage par distillation et mise en contact avec du solvant anhydre, de manière à produire d'une part un mélange gazeux sec (26), dont la teneur en hydrocarbures aromatiques, notamment benzène, est inférieure à 0,1 % en poids et d'autre part une coupe hydrocarbonée (27) renfermant la plus grande partie des hydrocarbures aromatiques contenus dans le mélange gazeux à traiter et un liquide (28) consistant en solvant renfermant de l'eau.

**Patentansprüche**

1. Tiefsttemperaturverfahren zur gleichzeitigen selektiven Entschwefelung und Abtrennung von Kohlenwasserstoffen aus einem Gasgemisch, das in der Hauptsache aus Methan besteht und außerdem $H_2S$, Kohlenwasserstoffe mit zwei und mehr C-Atomen und gegebenenfalls eine oder mehrere gasförmige Verbindungen enthält, ausgewählt unter den Inertgasen, $H_2O$, $CO_2$, COS und Merkaptanen, und dieses Gasgemisch sich unter einem absoluten Druck von über 0,5 MPa befindet, wobei man das Gasgemisch in einer Waschzone (5) mit einem Lösungsmittel (6) kontaktiert, das aus einer Flüssigkeit besteht,

die vorzugsweise $H_2S$, COS und die Merkaptane und lediglich einen Teil des $CO_2$ löst und einerseits eine Siedetemperatur bei Atmosphärendruck von über 40°C und andererseits eine Vikosität von unter 0,1 Pa.s bei -40°C aufweist, man das das $H_2S$ und die anderen absorbierten Verbindungen enthaltende Lösungsmittel regeneriert und das regenerierte Lösungsmittel dann wieder der Waschzone zuführt, dadurch gekennzeichnet, daß man die Kontaktierung des Gasgemisches mit dem Lösungsmittel bei einer ausreichend niedrigen Temperatur und bei einem solchen Verhältnis der Durchströmmenge des zu behandeldenden Gasgemisches zu der des Lösungsmittels und bei einer solchen Zahl theoretischer Waschböden durchführt, daß man einerseits ein behandeltes Gas (7, 64) erhält, das in der Hauptsache aus Methan besteht und dessen $H_2S$-Partialdruck unter 65 Pa liegt und, wenn das zu behandelnde Gasgemisch neben $H_2S$ auch noch COS und/oder Merkaptane enthält, der Gesamtpartialdruck der Schwefelverbindungen unter 260 Pa liegt, und andererseits eine als angereichertes Lösungsmittel (8) bezeichnete Flüssigphase, die aus einem mit $H_2S$ und anderen absorbierten Verbindungen angereicherten Lösungsmittel und einer Fraktion kondensierter Kohlenwasserstoffe, die wenigstens 80 Mol.% der im zu behandelnden Gasgemisch vorliegenden Kohlenwasserstoffe mit 3 und mehr C-Atomen ausmachen, zusammengesetzt ist, und daß man das angereicherte Lösungsmittel wenigstens einer teilweisen Entmethanung (9, 29) unterzieht, um eine an Methan verarmte, als entmethantes angereichertes Lösungsmittel (11) bezeichnete Flüssigphase und eine mit Methan angereicherte Gasphase (10) zu erzeugen, man das entmethante angereicherte Lösungsmittel auf eine Temperatur abkühlt (12), die in einem Maße unter der in der Waschzone herrschenden Temperatur liegt, daß es zu einer Entmischung des entmethanten Lösungsmittels zu einer als gereinigtes angereichertes Lösungsmittel (14, 38) bezeichneten unteren Flüssigfraktion, welche die durch das Lösungsmittel gelösten sauren Verbindungen $H_2S$ und gegebenenfalls $CO_2$, COS und Merkaptane enthält und einen Kohlenwasserstoffgehalt, ausgedrückt in Methanäquivalent, von unter 5 Mol.% der Menge der sauren Verbindungen aufweist, und zu einer oberen Flüssigfraktion (13, 37) kommt, die als Schwerkohlenwasserstoffprimärfraktion bezeichnet wird und aus dem Rest der Kohlenwasserstoffe gebildet wird, die im entmethanten angereicherten Lösungsmittel enthalten waren, man die Schwerkohlenwasserstofffraktion von dem gereinigten angereicherten Lösungsmittels abtrennt und man das gereinigte angereicherte Lösungsmittel einer Regenerationsbehandlung (46, 16, 20) unterzieht, um einen Strom (24) aus saurem Gas, der fast die gesamte Menge an $H_2S$ und der übrigen gegebenenfalls im gereinigten angereicherten Lösungsmittel enthaltenen sauren Verbindungen enthält und einen Kohlenwasserstoffgehalt, ausgedrückt in Methanäquivalent, von unter 5 Mol.%, bezogen auf die sauren Verbindungen aufweist, und das regenerierte Lösungsmittel (21), das in die Waschzone (5) zurückgeführt wird, zu erzeugen.

2. Verfahren nach Anspruch 1, dadurch gekennzeichnet, daß das mit dem zu behandelnden Gasgemisch in Berührung gebrachte Lösungsmittel eine Viskosität von unter 0,05 Pa.s aufweist.

3. Verfahren nach Anspruch 1 oder 2, dadurch gekennzeichnet, daß das mit dem zu behandelnden Gasgemisch in Berührung gebrachte Lösungsmittel aus einem oder mehreren flüssigen organischen Absorbentien, die in wasserfreier Form oder im Gemisch mit Wasser verwendet, besteht, wobei das oder die Absorbentien ausgewählt werden unter den

Amiden der Formeln 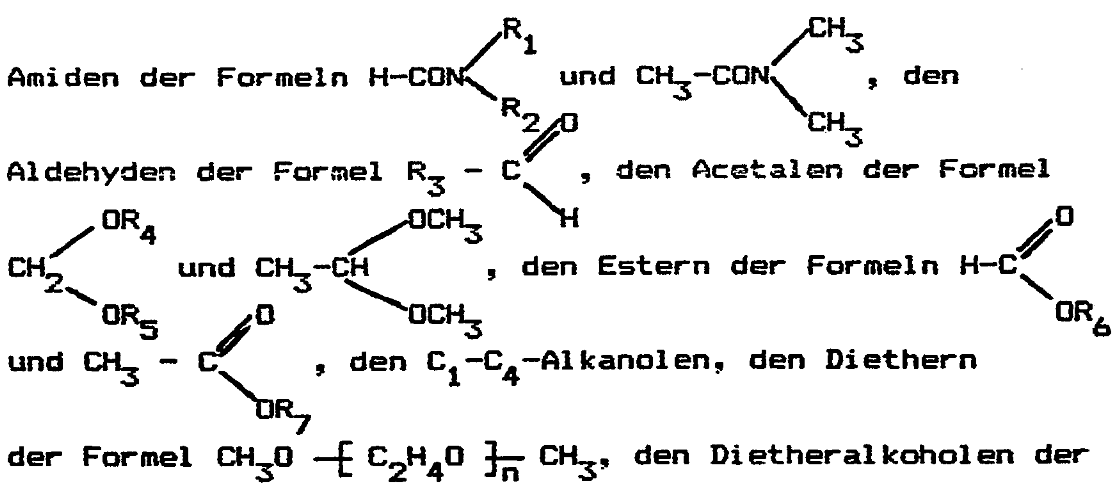 und , den Aldehyden der Formel , den Acetalen der Formel und , den Estern der Formeln und , den $C_1$-$C_4$-Alkanolen, den Diethern der Formel $CH_3O-[C_2H_4O]_n-CH_3$, den Dietheralkoholen der Formel $R_9O-C_2H_4-O-C_2H_4-OH$, den Laktonen der Formel

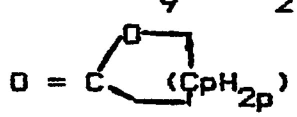

und Propylenkarbonat, wobei in diesen Formeln $R_1$ und $R_2$ gleich oder verschieden sind und ein Wasser-

stoffatom oder einen $C_1$-oder $C_2$-Alkylrest bedeuten, $R_3$ einen $C_3$ oder $C_4$-Alkylrest bedeutet, $R_4$ und $R_5$ gleich oder verschieden sind und einen bedeuten, $R_4$ einen $C_2$-$C_4$-Alkylrest oder einen Rest

$$\left[ C_2H_4O \right]_n - R_8$$

darstellt, in welchem $R_8$ einen $C_1$- oder $C_2$-Alkylrest und n 1 oder 2 bedeuten, $R_7$ einen $C_1$- oder $C_2$-Alkylrest oder einen Rest

$$\left[ C_2H_4O \right]_n - R_8$$

und $R_9$ einen $C_1$-$C_4$-Alkylrest darstellt und p eine ganze Zahl von 2 bis 4 bedeutet.

4. Verfahren nach einem der Ansprüche 1 bis 3, dadurch gekennzeichnet, daß die Temperatur für die Kontaktierung des zu behandelnden Gasgemisches mit dem Lösungsmittel in der Waschzone in einem Bereich zwischen 0 und –45°C liegt.

5. Verfahren nach einem der Ansprüche 1 bis 4, dadurch gekennzeichnet, daß die Waschzone, in der das zu behandelnde Gasgemisch mit dem Lösungsmittel kontaktiert wird, aus einer oder mehreren Waschkolonnen besteht, die jeweils mindestens ca. 10, vorzugsweise 20 theoretische Waschböden umfassen.

6. Verfahren nach Anspruch 5, dadurch gekennzeichnet, daß man die Temperatur in jeder Waschkolonne durch indirekten Wärmetausch (61), der an einem oder mehreren Punkten der in Frage kommenden Kolonne zwischen dem in dieser Kolonne enthaltenen flüssigen Medium und einer Kühlflüssigkeit durchgeführt wird, vorzugsweise mit einer Abweichung von 15°C, hält..

7. Verfahren nach einem der Ansprüche 1 bis 6, dadurch gekennzeichnet, daß das zu behandelnde Gasgemisch vor seiner Kontaktierung mit dem Lösungsmittel einer Abkühlung (2) auf eine Temperatur zwischen 0 und –30°C unterworfen wird, um einerseits eine als Kondensate (4) bezeichnete Flüssigphase, die in erster Linie Kohlenwasserstoffe mit 3 und mehr C-Atomen und $H_2S$ enthält, und andererseits eine als vorbehandeltes Gasgemisch (3) bezeichnete Gasphase zu erzeugen, man dann das vorbehandelte Gasgemisch mit dem Lösungsmittel in der Waschzone (5) kontaktiert und das aus der Waschzone austretende Lösungsmittel (8) mit den aus der Abkühlungsstufe austretenden Kondensaten (4) vereinigt, um das angereicherte Lösungsmittel (9a) zu bilden, das man dann der Entmethanungsbehandlung unterzieht.

8. Verfahren nach einem der Ansprüche 1 bis 6, dadurch gekennzeichnet, daß das zu behandelnde Gasgemisch vor seiner Kontaktierung mit dem Lösungsmittel einer Abkühlung (2) auf eine Temperatur zwischen 0 und –30°C unterworfen wird, um einerseits eine als Kondensate (4) bezeichnete Flüssigphase, die in erster Linie Kohlenwasserstoffe mit 3 und mehr C-Atomen und $H_2S$ enthält, und andererseits eine als vorbehandeltes Gasgemisch (3) bezeichnete Gasphase zu erzeugen, man dann das vorbehandelte Gasgemisch mit dem Lösungsmittel in der Waschzone (5) kontaktiert und jede der Flüssigphasen, die aus dem aus der Waschzone stammenden Lösungsmittel (8) und den aus der Kühlstufe (2) stammenden Kondensaten (4) bestehen, einer getrennten Entmethanungsbehandlung unterzieht, wobei die im Laufe der Entmethanungsbehandlungen erzeugten Gasphasen getrennt oder im Gemisch dem zu behandelnden Gasgemisch (1) oberhalb der Kühlstufe (2) gegebenenfalls wieder zugeführt wurden und man das entmethante Lösungsmittel mit den entmethanten Kondensaten vereinigt, um das entmethante angereicherte Lösungsmittel (11) zu bilden.

9. Verfahren nach einem der Ansprüche 1 bis 8, dadurch gekennzeichnet, daß die auf das angereicherte Lösungsmittel und die Kondensate angewandte Entmethanungsbehandlung in zwei Stufen durchgeführt wird, das heißt in einer ersten Stufe, auf der die zu entmethanende Flüssigkeit, das heißt das angereicherte Lösungsmittel bzw. die Kondensate einer ersten Entspannung (29) bei einem mittleren Druck unterzogen werden, der geeignet ist, eine erhebliche Menge des in der zu entmethanenden Flüssigkeit gelösten Methans freizusetzen und ein erstes mit Methan angereichertes Gas (34) und eine vorentmethante Flüssigkeit (35) zu erzeugen, und in einer zweiten Stufe, auf der die vorentmethante Flüssigkeit einer zweiten Entspannung und dann einer Destillation (9) unterworfen wird, wodurch ein zweiter mit Methan angereicherter Strom (32) und eine entmethante Flüssigkeit (11) gebildet werden, wobei das zweite mit Methan angereicherte Gas bis zum Druck des ersten mit Methan angereicherten Gases komprimiert und dann mit diesem gemischt wird, um die mit Methan angereicherte Gasphase (10) zu bilden.

10. Verfahren nach Anspruch 9, dadurch gekennzeichnet, daß auf der zweiten Stufe der Entmethanungsbehandlung die Destillation (9) nach der zweiten Entspannung durch erneute Erwärmung in mindestens zwei in Serie geschalteten Erwärmungszonen (30a, 30b) mit Hilfe einer Flüssigkeit (31) durchgeführt wird, die entweder aus wenigstens einem Teil des zu behandelnden Gasgemisches, der dem Gasgemisch vor seiner Kontaktierung mit dem Lösungsmittel oder vor seiner Abkühlung entnommen wurde, oder außerdem auch noch aus einem Teil des regenerierten Lösungsmittels besteht.

11. Verfahren nach einem der Ansprüche 1 bis 10, dadurch gekennzeichnet, daß die Temperatur, die unterhalb der Temperatur des entmethanten angereicherten Lösungsmittels liegt, bei der man das Lösungsmittel zur Auslösung seiner Entmischung abkühlt (12), zwischen -25 und -80°C liegt.

12. Verfahren nach einem der Ansprüche 1 bis 11, dadurch gekennzeichnet, daß die Regenerationsbehandlung des gereinigten angereicherten Lösungsmittels eine Stufe der teilweisen Regeneration umfaßt, die eine Entspannung (15) des gereinigten angereicherten Lösungsmittels bis auf einen Druck von über 100 KPa und vorzugsweise zwischen 150 und 300 KPa umfaßt, gefolgt von wenigstens einer teilweisen Verdampfung (46, 16) des entspannten Lösungsmittels, wodurch einerseits wenigstens eine Fraktion (47, 17) des sauren Gases und andererseits ein halbregeneriertes Lösungsmittel (18) erzeugt werden, dessen Temperatur zumindest 0°C beträgt und dessen Druck, der unterhalb des Drucks des entspannten Lösungsmittels liegt, wenigstens 100 KPa beträgt, ferner eine Stufe der vollständigen Regeneration des halbregenerierten Lösungsmittels durch Destillation (20), insbesondere durch erneute Erwärmung, wodurch eine Fraktion aus saurem Gas (19) und dem regenerierten Lösungsmittel (21) entsteht, wobei dieses nach entsprechender Abkühlung (22) wieder der Waschzone (5) zugeführt (6) wird, während die einzelnen Fraktionen von saurem Gas (47, 17, 19) miteinander vereinigt werden, um den Strom an saurem Gas (24) zu bilden, dessen Gehalt an Kohlenwasserstoffen, ausgedrückt in Methanäquivalent, unter 5 Mol.% der sauren Verbindungen beträgt.

13. Verfahren nach Anspruch 12, dadurch gekennzeichnet, daß auf der Stufe der Teilregeneration des gereinigten angereicherten Lösungsmittels die teilweise Verdampfung des entspannten Lösungsmittels durch eine vorausgehende erste teilweise Verdampfung (46) des Lösungsmittels unter Gewinnung einer ersten Sauergasfraktion (47) und einer ersten Flüssigphase (50) mit einer Temperatur unter 0°C und eine nachfolgende zweite teilweise Verdampfung (16) der ersten Flüssigphase unter Gewinnung einer zweiten Sauergasfraktion (17) und einer zweiten Flüssigphase mit einer Temperatur von mindestens 0°C und einem Druck, der unterhalb des Drucks des entspannten Lösungsmittels liegt und wenigstens 100 KPa beträgt, durchgeführt wird, wobei die zweite Flüssigphase das auf der Stufe der Totalregeneration zu behandelnde halbregenerierte Lösungsmittel bildet und wobei die erste Sauergasfraktion (47) nach ihrer Wiedererwärmung (49) auf 0°C oder darüber mit der zweiten Sauergasfraktion (17) und der Sauergasfraktion (19) vereinigt wird, die sich aus der Totalregeneration des halbregenerierten Lösungsmittels ergibt, um den Sauergasstrom (24) zu bilden, dessen Kohlenwasserstoffgehalt, ausgedrückt in Methanäquivalent, unter 5 Mol.% der sauren Verbindungen beträgt.

14. Verfahren nach Anspruch 12 oder 13, dadurch gekennzeichnet, daß die Totalregeneration des halbregenerierten Lösungsmittels durch Destillation in einer Kolonne (20) durchgeführt wird, die eine Vielzahl von theoretischen Böden umfaßt, wobei das regenerierte Lösungsmittel am Boden der Kolonne abgezogen (21) wird, und das halbregenerierte Lösungsmittel in zwei Ströme aufgeteilt wird, von denen einer (51) dem Kopfboden der Destillationskolonne zugeführt wird und der andere (52) nach Wiedererwärmung im Gegenstrom (52a) mit dem aus der Kolonne abgezogenen regenerierten Lösungsmittel diese auf der Höhe eines mittleren theoretischen Bodens speist.

15. Verfahren nach einem der Ansprüche 1 bis 14, dadurch gekennzeichnet, daß das gereinigte angereicherte Lösungsmittel (11) vor seiner Regeneration einer zusätzlichen Behandlung zur Entfernung der Kohlenwasserstoffe unterzogen wird, indem man das Lösungsmittel in einer ersten Flüssig-Flüssig-Extraktionszone (39), die vorzugsweise wenigsten 4 theoretische Extraktionsböden umfaßt, mit einem Extraktionsmittel kontaktiert, das in der Hauptsache aus Kohlenwasserstoffen besteht, deren Molekularmasse über der des n-Pentans liegt, um aus dem gereinigten angereicherten Lösungsmittel eine zweite Flüssigfraktion (62) von Kohlenwasserstoffen abzutrennen, diese zweite Fraktion (62) mit der ersten Schwerkohlenwasserstofffraktion (37) vereinigt, das auf diese Weise erhaltene Gemisch wieder erwärmt (41) und destilliert (42), um die Schwerkohlenwasserstofffraktion (13) zu erhalten, die wenigstens 80 Mol.% der in dem zu behandelnden Gasgemisch vorliegenden Kohlenstoffe mit 3 und mehr C-Atomen enthält, und das Extraktionsmittel (43) zurückzugewinnen, dessen Hauptteil nach Abkühlung (44) bis auf eine Temperatur, die im wesentlichen derjenigen entspricht, die bei der Entmischung des entmethanten angereicherten Lösungsmittels erreicht wurde, der ersten Flüssig-Flüssig-Extraktionszone (39) wieder zugeführt (40) wird.

16. Verfahren nach einem der Ansprüche 1 bis 15, dadurch gekennzeichnet, daß das aus der Waschzone austretende behandelte Gas auf wenigstens 15°C abgekühlt (55) wird, um ein abgekühltes behandeltes Gas (58) und eine Flüssigfraktion (57) zu erzeugen, die im Gemisch mit dem angereicherten Lösungsmittel (9a) einer Entmethanung unterzogen wird.

17. Verfahren nach Anspruch 15 und 16 dadurch gekennzeichnet, daß man das abgekühlte behandelte Gas in einer zusätzlichen Extraktionszone (60) mit Hilfe des in der ersten Extraktionszone (39) nicht verwendeten Teils (45) des Extraktionsmittels einer zusätzlichen Extraktion unterzieht, um ein behandeltes Gas (7) von erhöhter Reinheit und eine Flüssigfraktion (61) zu erzeugen, die man wieder der Destillation (41, 42) des Gemischs der ersten und zweiten Kohlenwasserstofffraktion zuführt.

18. Verfahren nach einem der Ansprüche 1 bis 17, dadurch gekennzeichnet, daß das zu behandelnde Gasgemisch vor jeglicher Behandlung einem Vorgang (25) der Trocknung und Entbenzolung durch Destillation und Kontaktierung mit dem wasserfreien Lösungsmittel unterzogen wird, um einerseits ein trockenes Gasgemisch (26), dessen Gehalt an aromatischen Kohlenwasserstoffen, insbesondere an Benzol, unter 0,1 Gew.-% liegt, und andererseits eine Kohlenwasserstofffraktion (27) zu erhalten, die

den größten Teil der in dem zu behandelnden Gasgemisch enthaltenen aromatischen Kohlenwasserstoffe enthält, sowie eine Flüssigkeit (28), die aus dem Wasser enthaltenden Lösungsmittel besteht, zu erzeugen.

## Claims

1. Cryogenic process for the simultaneous selective desulphurisation and degazolinage of a gaseous mixture principally consisting of methane and also containing $H_2S$ and hydrocarbons containing at least two carbon atoms and possibly one or a plurality of gaseous compounds selected from the inert gases, $H_2O$, $CO_2$, COS and mercaptans, this gaseous mixture being at an absolute pressure greater than 0.5 MPa, the said process being of the type in which the gaseous mixture, in a washing area (5), is brough into contact with a solvent (6) consisting of a liquid which preferably dissolves $H_2S$, COS and the mercaptans and only part of the $CO_2$ and which has, on the one hand, a boiling temperature at atmospneric pressure greater than 40°C and, on the other hand, has a viscosity at -40° C of less than 0.1 Pa.s, and the solvent containing the $H_2S$ and the other absorbed compounds is regenerated then the regenerated solvent is recycled towards the washing zone, and characterised in that the said mixing of the gaseous mixture and the solvent occurs at a temperature which is sufficiently low and with a ratio of mass flows of the gaseous mixture to be treated and solvent and a number of theoretical washing stages such that, on the one hand, a treated gas (7, 64) which principally consists of methane and of which the partial pressure of $H_2S$ is less than 65 Pa and, when the gaseous mixture to be treated also comprises COS and/or mercaptans in addition to $H_2S$, the global partial pressure of the sulphurous compounds being less than 260 Pa and, on the other hand, a liquid phase known as a rich solvent (8) and consisting of the solvent enriched with $H_2S$ and other absorbed compounds and a fraction of condensed hydrocarbons representing at least 80 mol percent of the hydrocarbons containing at least three carbon atoms which were present in the gaseous mixture to be treated; and in that the rich solvent is subjected to an at least partial demethanisation treatment (9, 29) in order to produce a liquid phase depleted of methane and known as demethanised rich solvent (11) and a gaseous phase (10) rich in methane, the demethanised rich solvent is cooled (12) to a temperature sufficiently lower than the temperature prevailing in the washing zone in order to produce a segregation of the said demethanised solvent into a lower liquid fraction known as purified rich solvent (14, 38), which comprises the $H_2S$ acid compounds and if necessary $CO_2$, COS and mercaptans dissolved by the solvent, and having a content of hydrocarbons, expressed as a methane equivalent, of less than 5 mol percent of the acid compound quantity, and a superior liquid fraction (13, 37) known as the primary fraction of heavy hydrocarbons and formed from the remainder of the hydrocarbons which were present in the demethanised rich solvent, the heavy hydrocarbon fraction is separated from the purified rich solvent and the said purified rich solvent is subjected to the regeneration process (46, 16, 20) in order to produce an acid gas stream (24) comprising almost all the $H_2S$ and other acid compounds possibly present in the purified rich solvent and containing less than 5 mol percent of hydrocarbons, expressed as a methane equivalent, in relation to the acid compounds and regenerated solvent (21) which is recycled (6) towards the washing zone (5).

2. Process according to Claim 1, characterised in that the solvent mixed with the gaseous mixture to be treated has a viscosity of less than 0.05 Pa.s.

3. Process according to Claim 1 or 2, characterised in that the solvent mixed with the gaseous mixture to be treated consists of one or a plurality of liquid organic absorbents used in the anhydrous form or mixed with water, the said absorbent or absorbents being selected

from the amides having the formula 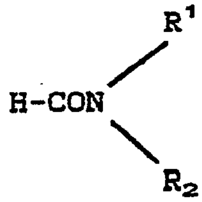 and 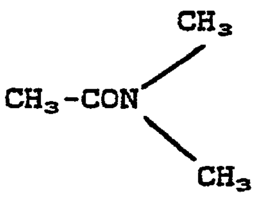, aldehydes having the formula 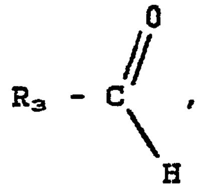, acetals having the formula 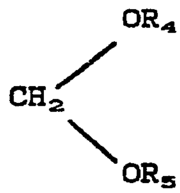 and 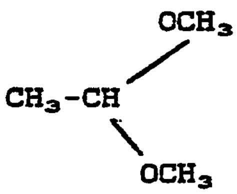, esters having the formula 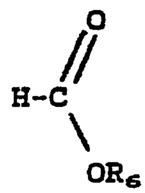 and 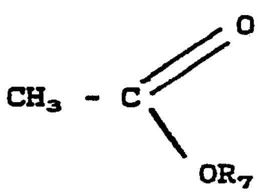, $C_1$ to $C_4$ alkanols, diethers having the formula $CH_3O-[C_2H_4]_n-CH_3$, alcohol diethers having the formula $R_9O-C_2H_4-O-C_2H_4-OH$, lactones having the formula

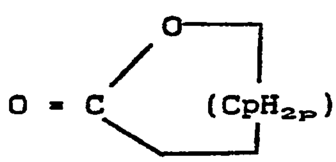

and propylene carbonate, with $R_1$ and $R_2$ in these formulae designating a hydrogen atom or a $C_1$ or $C_2$ alkyl radical, $R_3$ being a $C_3$ or $C_4$ alkyl radical, $R_4$ and $R_5$, identical or different, representing a $C_1$ to $C_3$ alkyl radical, $R_6$ being a $C_2$ to $C_4$ alkyl radical or a

$$[C_2H_4O]_n-R_8$$

radical with $R_8$ designating a $C_1$ or $C_2$ alkyl radical and n being 1 or 2, $R_7$ being a $C_1$ or $C_2$ alkyl radical or a

$$\left[C_2H_4O\right]_n - R_8$$

radical, $R_9$ designating a $C_1$ to $C_4$ alkyl radical and p being an integer between 2 and 4.

4. Process according to any one of Claims 1 to 3, characterised in that the temperature for mixing th gaseous mixture to be processed with the solvent, in the washing zone, is between 0° C and –45°C.

5. Process according to any one of Claims 1 to 4, characterised in that the washing zone in which the gaseous mixture to be treated is brought into contact with the solvent, consists of one or a plurality of washing columns, each containing at least approximately 10 and preferably at least 20 theoretical washing stages.

6. Process according to Claim 5, characterised in that the temperature is maintained in each of the washing columns preferably to within + or –5°C approximately, by indirect heat exchange (61) performed at one or a plurality of points on the column in question, between the fluid medium contained in this column and a refrigerant.

7. Process according to any one of Claims 1 to 6, characterised in that, prior to its being brought into contact with the solvent, the gaseous mixture to be treated is subjected to refrigeration (2) to a temperature between 0°C and –30°C in order to produce, on the one hand, a liquid phase known as condensates (4) principally containing hydrocarbons having at least three carbon atoms and $H_2S$ and, on the other hand, a gaseous phase known as the pretreated gaseous mixture (3), then the said pre-treated gaseous mixture is brought into contact with the solvent in the washing zone (S) and the solvent (8) from the washing zone is combined with the condensates (4) from the refrigeration stage in order to form a rich solvent (9a) which is subjected to the demethanisation treatment.

8. Process according to any one of Claims 1 to 6, characterised in that prior to its being brought into contact with the solvent, the gaseous mixture to be treated is refrigerated (2) to a temperature between 0° and –30°C in order to produce, on the one hand, a liquid, phase known as condensates (4) principally containing hydrocarbons having at least three carbon atoms and $H_2S$ and, on the other hand, a gaseous phase known as the pre-treated gaseous mixture (3), then the said pre-treated gaseous mixture is brought into contact with the solvent in the washing zone (S) and each of the liquid phases consisting of the solvent (8) from the washing zone and the condensates (4) from the refrigeration stage (2) are subjected to a separate demethanisation treatment, the gaseous phases produced during the demethanisation treatments possibly being recycled, separately or in a mixture, towards the gaseous mixture (1) to be treated upstream of the refrigeration stage (2), and the solvent and condensates from the separate demethanisation treatments are combined in order to form the demethanised rich solvent (11).

9. Process according to any one of Claims 1 to 8, characterised in that the demethanisation treatment applied to the rich solvent and the condensates is performed in two stages, ie. a first stage in which the fluid to be demethanised, that is the rich solvent or condensates, is subjected to a first expansion process (29) at an intermediate pressure suitable for releasing a large fraction of the methane dissolved in the said fluid to be demethanised and for producing a first methane-rich gas (34) and a pre-demethanised fluid (35), and a second stage in which the pre-demethanised fluid is subjected to a second expansion stage then to distillation (9) so as to produce a second methane-rich gas (32) and a demethanised fluid (11), the second methane-rich gas being compressed to the pressure of the first methane-rich gas then mixed therewith in order to form the methane-rich gaseous phase (10).

10. Process according to Claim 9, characterised in that, in the second demethanisation treatment stage, the distillation (9) following the second expansion stage is performed by boiling off in at least two boiling-off zones (30a, 30b) arranged in series, by means of one fluid (31) consisting either of at least some of the gaseous mixture to be treated, taken from the said gaseous mixture before it is brought into contact with the solvent, or before its refrigeration, or even of some of the regenerated solvent.

11. Process according to any one of Claims 1 to 10, characterised in that the temperature, lower than the temperature of the demethanised rich solvent, to which the said solvent is cooled (12) in order to cause the segregation thereof is between –25°C and –80°C.

12. Process according to any one of Claims 1 to 11, characterised in that the regeneration treatment of the purified rich solvent comprises a partial regeneration stage comprising expansion (15) of the purified rich solvent to a pressure greater than 100 KPa and preferably between 150 KPa and 300 KPa followed by at least one partial vaporisation process (46, 16) of the said expanded solvent so as to produce, on the one hand, at least one fraction (47, 17) of acid gas and, on the other hand, a semi-regenerated solvent (18) of which the temperature is at least equal to 0°C and the pressure, lower than the pressure of the expanded solvent, is at least 100 KPa, then a total regeneration stage for the semi-regenerated solvent by distillation (20), in particular using a boiling-off process and producing an acid gas fraction (19) and regenerated solvent fraction (21), the said regenerated solvent being recycled (6), after suitable cooling (22), towards the washing zone (5) whilst the different acid gas fractions (47, 17, 19) are combined to form the acid gas current (24)1 the hydrocarbon content of which, expressed as a methane equivalent, is less than 5 mol percent of the acid compounds.

13. Process according to Claim 12, characterised in that in the partial regeneration stage of the purified rich solvent, the partial vaporisation of the expanded solvent is performed firstly by a primary partial vaporisation (46) of the said solvent so as to produce an acid gas primary fraction (47) and a primary liquid phase (50), of which the temperature is less than 0°C, then by performing a secondary partial vaporisation (16) of the primary liquid phase in order to produce an acid gas secondary fraction (17) and a secondary liquid phase (18) having a temperature of at least 0°C and a pressure, less than the pressure of the expanded solvent, of at least 100 KPa, the said secondary liquid phase constituting the semi-regenerated solvent which is treated in the total regeneration stage, the acid gas primary fraction (47) being reheated (49) to a temperature equal to or greater than 0°C then combined with the acid gas secondary fraction (17) and the acid gas fraction (19) resulting from the total regeneration of the semi-regenerated solvent in order to form the acid gas current (24), of which the hydrocarbon content, expressed as a methane equivalent, is less than 5 mol percent of the acid compounds.

14. Process according to Claim 12 or 13, characterised in that as the total regeneration of the semi-regenerated solvent by distillation is performed in a column (20) comprising a plurality of theoretical stages with the regenerated solvent being drawn off (21) at the base of the column, the semi-regenerated solvent is divided into two streams, of which one (51) is conveyed to the plate head of the distillation column and the other (52) supplies the said column at the level of a theoretical intermediate stage, after reheating (52a) in a counter flow manner with the regenerated solvent drawn off from the column.

15. Process according to any one of Claims 1 to 14, characterised in that, before its regeneration, the purified rich solvent (11) is subjected to a complementary hydrocarbon elimination treatment, the said treatment consisting of bringing the said solvent in a liquid-to-liquid primary extraction zone (39), preferably comprising at least four theoretical extraction stages, into contact with an extraction agent principally consisting of hydrocarbons, of which the mol mass is greater than that of n-pentane, in order to separate a hydrocarbon liquid secondary fraction (62) from the said purified rich solvent, combining the said secondary fraction (62) with the heavy hydrocarbon primary fraction (37), then reheating (41) and distilling (42) the mixture thus obtained in order to produce the heavy hydrocarbon fraction (13) containing at least 80 mol percent of the hydrocarbons having at least three carbon atoms which were present in the gaseous mixture to be treated, and recovering the extraction agent (43), of which the main part is recycled (40) towards the liquid-to-liquid primary extraction zone (39), after cooling (44) to a temperature substantially equal to the temperature reached when the demethanised rich solvent is segregated.

16. Process according to any one of Claims 1 to 15, characterised in that the treated gas coming from the washing zone is refrigerated (55) by at least 15°C so as to produce a refrigerated treated gas (58) and a liquid fraction (57) which is subjected to the demethanisation treatment in a mixture with the rich solvent (9a).

17. Process according to Claims 15 and 16, characterised in that, in a complementary extraction zone (60), the refrigerated treated gas is subjected to an additional extraction process by means of the extraction agent part (45) not used in the primary extraction zone (39) so as to produce a treated gas (7) having an improved degree of purity and a liquid fraction (61) which is recycled towards the distillation process (41, 42) of the hydrocarbon primary and secondary fraction mixture.

18. Process according to any one of Claims 1 to 17, characterised in that before any treatment, the gaseous mixture to be treated is subjected to a drying and benzol stripping operation (25) by distillation and bringing into contact with some anhydrous solvent, so as to produce, on the one hand, a dry gaseous mixture (26), of which the aromatic hydrocarbon content, in particular benzene, is less than 0.1 weight percent and, on the other hand, a hydrocarbon fraction (27) comprising the largest part of the aromatic hydrocarbons contained in the gaseous mixture to be treated and a liquid (28) consisting of the solvent which comprises water.

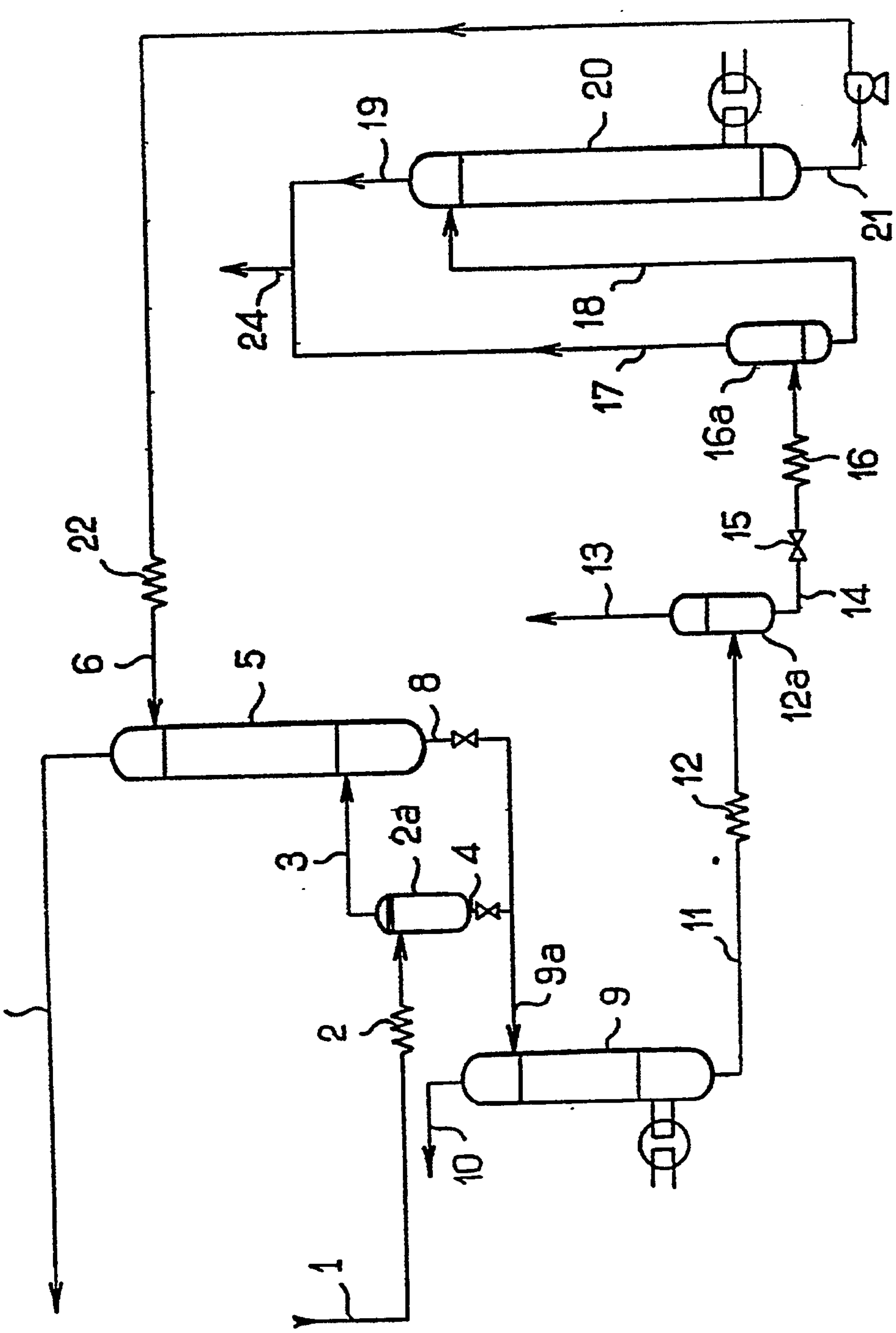

FIG_1

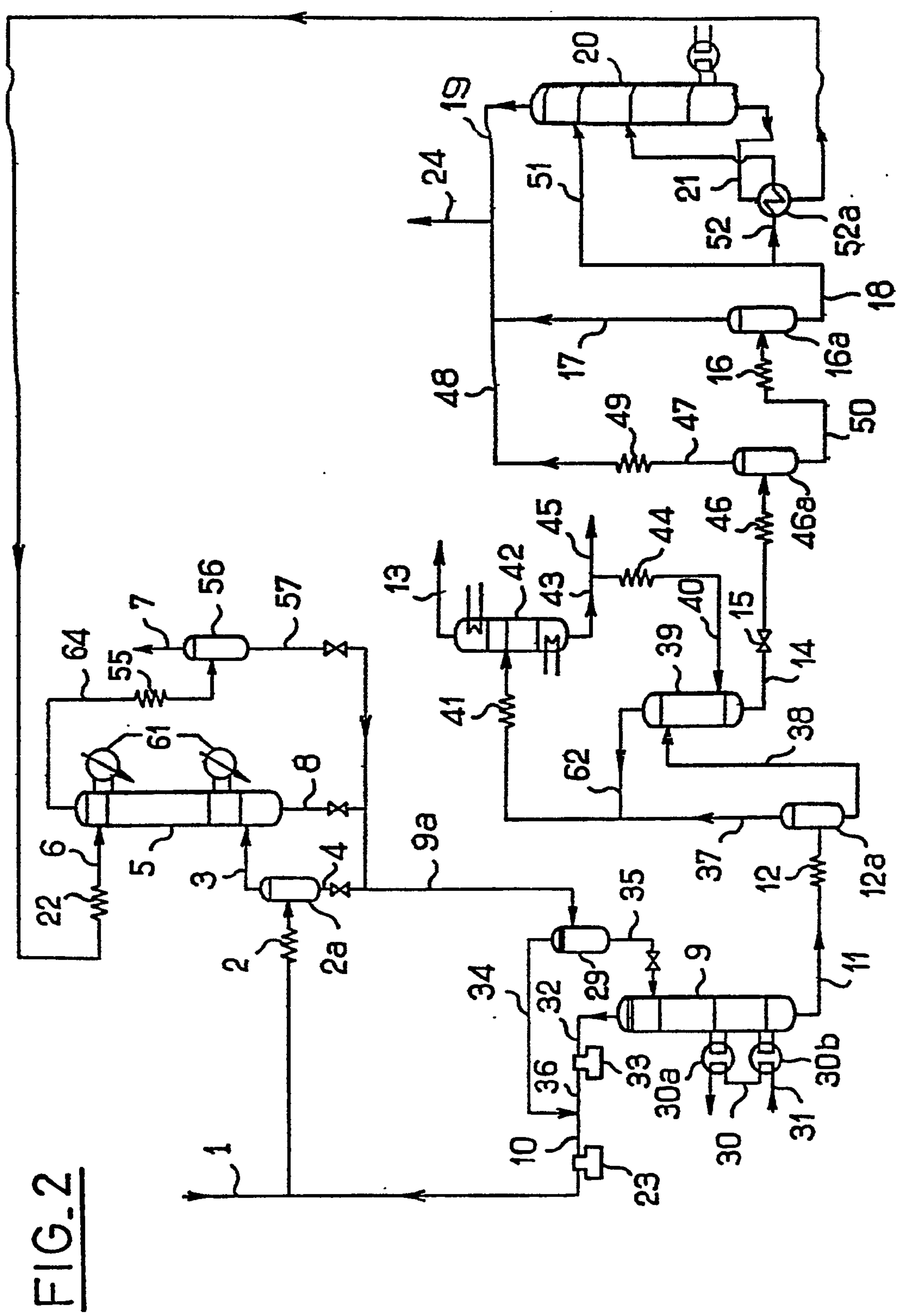
1
2
2a
3
4
5
6
7
8
9
9a
10
11
12
12a
13
14
15
16
16a
17
18
19
20
21
22
23
24
29
30
30a
30b
31
32
33
34
35
36
37
38
39
40
41
42
43
44
45
46
46a
47
48
49
50
51
52
52a
55
56
57
61
62
64

FIG. 2

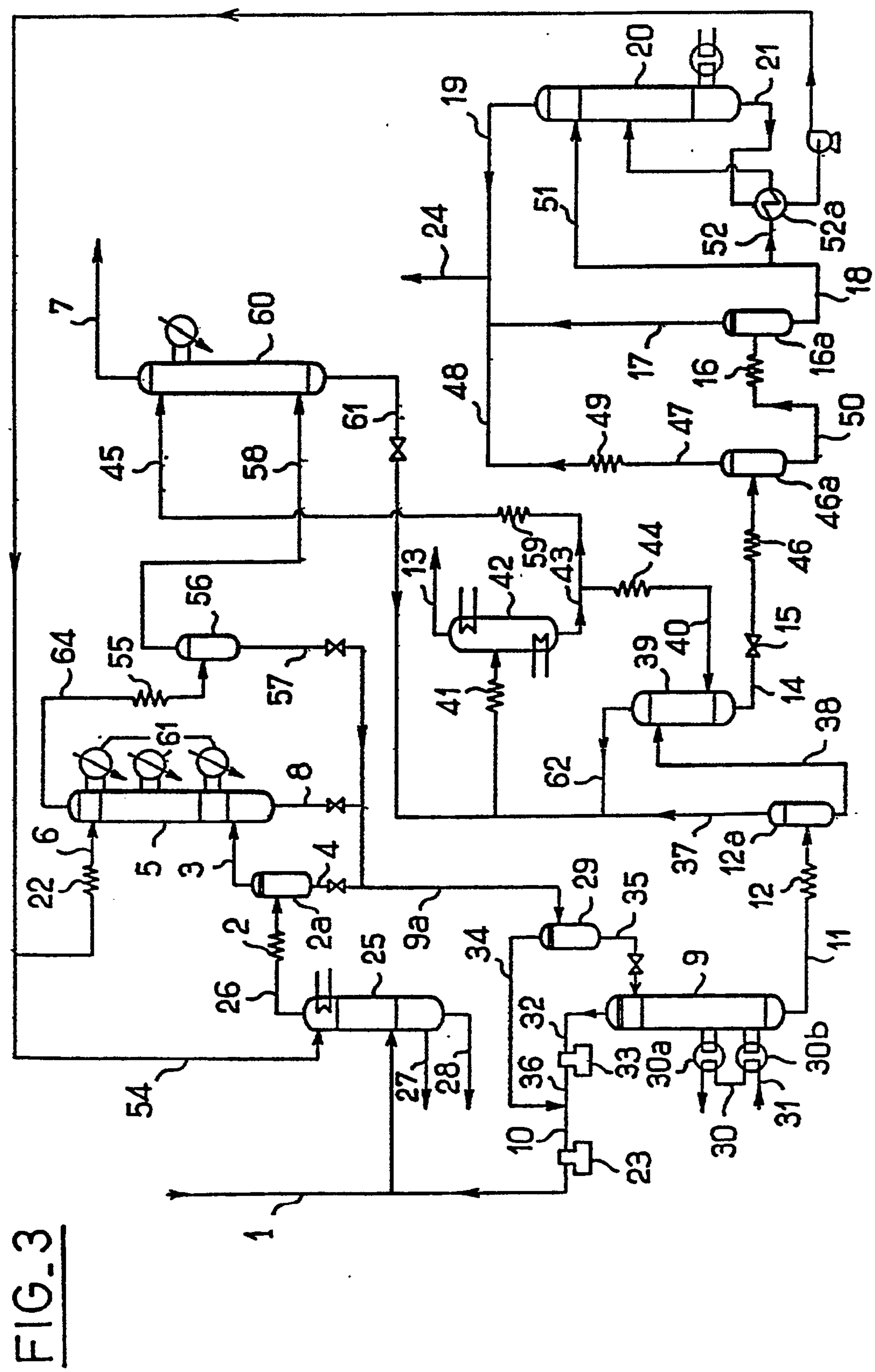

1
2
2a
3
4
5
6
7
8
9
9a
10
11
12
12a
13
14
15
16
16a
17
18
19
20
21
22
23
24
25
26
27
28
29
30
30a
30b
31
32
33
34
35
36
37
38
39
40
41
42
43
44
45
46
46a
47
48
49
50
51
52
52a
54
55
56
57
58
59
60
61
62
64


FIG_3

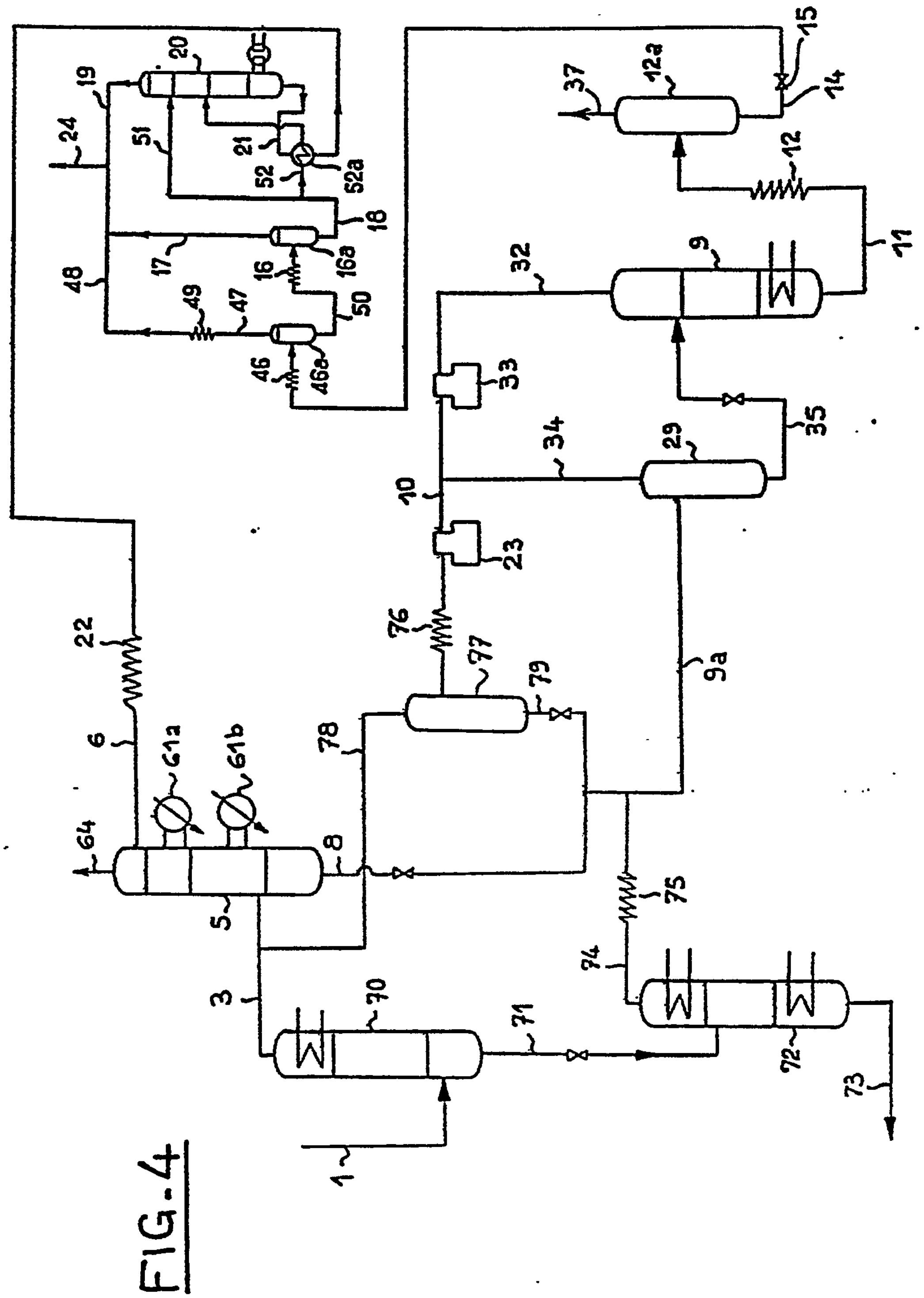
1
3
5
6
8
9
9a
10
11
12
12a
14
15
16
16a
17
18
19
20
21
22
23
24
29
32
33
34
35
37
46
46a
47
48
49
50
51
52
52a
61a
61b
64
70
71
72
73
74
75
76
77
78
79

FIG. 4